# EUROPEAN PATENT SPECIFICATION

(11) **EP 1 395 227 B1**
(45) Date of publication and mention of the grant of the patent: **28.05.2008**
(21) Application number: 02746437.9
(22) Date of filing: 23.05.2002
(51) Int. Cl.: C12Q 1/48

(54) **METHODS AND COMPOUNDS FOR THE DIAGNOSIS OF INFLAMMATORY DISEASE AND IDENTIFICATION OF PHARMACOLOGICAL AGENTS USEFUL IN THE TREATMENT OF INFLAMMATORY DISEASE**
VERFAHREN UND VERBINDUNGEN ZUR DIAGNOSE VON ENTZÜNDLICHEN ERKRANKUNGEN UND IDENTIFIZIERUNG VON PHARMAKOLOGISCHEN MITTELN ZUR BEHANDLUNG VON ENTZÜNDLICHEN ERKRANKUNGEN
METHODES ET COMPOSES UTILISES DANS LE DIAGNOSTIC D'UNE MALADIE INFLAMMATOIRE ET DANS L'IDENTIFICATION D'AGENTS PHARMACEUTIQUES UTILES POUR LE TRAITEMENT DE MALADIES INFLAMMATOIRES

(30) Priority: 23.05.2001 US 292968 P; 15.11.2001 US 335474 P; 28.11.2001 US 333848 P
(43) Date of publication of application: 10.03.2004
(73) Proprietor: Boehringer Ingelheim Pharmaceuticals Inc., Ridgefield, Connecticut 06877-0368 (US)
(72) Inventor: LI, Jun, Danbury, CT 06811 (US); LI, Xiang, John, Danbury, CT 06810 (US); BARTON, Randall, Farmington, CT 06032 (US)
(74) Representative: Mahlbacher, Volker
(86) International application number: PCT/US2002/016276
(87) International publication number: WO 2002/094195

(56) References cited:
- WO-A-02/12338
- US-A- 6 033 873
- US-A- 6 159 716
- DATABASE EMBL [Online] 25 December 1996 (1996-12-25), "Human pim-2 protooncogene homolog pim-2h mRNA, complete cds." XP002347146 retrieved from EBI accession no. EM_HUM:U77735 Database accession no. U77735

## Description

### BACKGROUND OF THE INVENTION

### 1. Field of the Invention

The field of this invention relates to the area of molecular biology. In particular, the present invention relates to the polynucleotide sequence encoding human Pim-2 (h-Pim-2) and the corresponding translated h-Pim-2 polypeptide, recombinant vectors comprising h-Pim-2 nucleic acid sequence, and methods for recombinant production of h-Pim-2 polypeptides, as well as the use of the same in diagnosing inflammatory disease states and in screening assays for identification of compositions that may be useful in the treatment of inflammatory disease states, in particular inflammatory bowel diseases such as ulcerative colitis and Crohn's Disease.

### 2. The Related Art

Pim-2 is a highly conserved serine/threonine kinase involved in cell proliferation, meiosis and the prevention of apoptosis (Baytel et al., Biochim. Biophys. Acta Gene Struct. Expr. 1442: 274 (1998)). *Pim-2* of mice, also known at *Tic-1,* has been reported to be about 53% identical in sequence at the amino acid level to the proto-oncogene *Pim-1,* and to be expressed at low levels in a variety of tissues, with the highest expression in the brain and thymus (van der Lugt et al., EMBO J. 14(11): 2536 (1995)). Like *Pim-1,* the *Pim-2* locus is also a common site of provirus integration (Haupt et al., Cell 65: 753 (1991); Bruer et al., Embo J. 8: 743 (1989)). In fact, *Pim-2* was first identified by means of proviral tagging experiments carried out in mice, and analysis of DNA obtained from outgrown tumors obtained after transplantation of primary lymphomas induced by inoculation of newborn BABL/c or C57BL10 mice in which the *Pim-1* gene was largely deleted by gene targeting with Moloney MuLV. (Breuer et al., Embo J. 8(3): 743 (1989)). Such studies suggest that *Pim-2* is a proviral integration site that carries somatically acquired proviruses in the majority of transplanted tumors (*Id*.).

The *Pim-1* proto-oncogene is believed to be one of the most potent collaborators of myc proto-oncogenes in inducing lymphomagenesis in mice (van der Lugt et al., EMBO J. 14(11): 2536 (1995)). Allen et al. (Oncogene 15: 1133 (1997)) suggest, based on proviral tagging experiments, that *Pim-2* is similar in oncogenic behavior to *Pim-1.* They note that while basal expression of *Pim-1* and *Pim-2* differ with respect to basal expression in tissues, that both genes are highly expressed in response to the same cytokines. A *Pim-2* transgene in lymphoid cells was seen to predispose mice to T-cell lymphomas like those promoted by *pim-*1 transgenes.

As iterated above, *Pim-2,* as the related *Pim-1* gene, encodes labile, cytoplasmic serine/threonine kinases. Phosphorylation of protein substrates by serine/threonine kinases is often involved in the transduction of signals from the cell surface receptors to intracellular effectors. It is believed that *Pim-2,* like *Pim-1,* is a target for gp130-mediated signal transducer and transcriptional activator 3 ("STAT3") signaling. As is known to those of ordinary skill in the art, the activation of STAT3 by the cytokine receptor gp130 is required for both G1 to S cell cycle transition, as well as, anti-apoptosis (Shirogane et al., Immunity 11: 709 (1999)).

Baytel et al. (Biochim. Biophys. Acta Gene Struct. Expr. 1442: 274 (1998)) report cloning of the h-*Pim-2* gene. In comparison to mouse *Pim-2,* h-*Pim-2* is reported by Baytel *et al.* to encode a protein that shares 90% identity and 93% similarity at the primary structure level. At the RNA level, two *Pim-2* transcripts have been identified in humans, a 2.2 kb transcript that is highly expressed in hematopoietic tissues and in leukemic and lymphoma cell lines, and a 5.0 kb transcript that is detectable in spleen, thymus, small intestine and colon apoptosis (Baytel et al., Biochim. Biophys. Acta Gene Struct. Expr. 1442: 274 (1998)). The *Pim-2* gene in humans is believed to be X-linked (van der Lugt et al., EMBO J. 14(11): 2536 (1995)).

The present inventors (Li et al., J. Biol. Chem. 276: 18579 (2001)) have recently disclosed that *Pim-2* is induced by lipopolysaccharide (LPS) in a variety of cell lines. Studies undertaken by the inventors suggest that up-regulation of *Pim-2* in 70Z3 cells by LPS is controlled by the IKK/NF-κB pathway.

Aberrant protein serine/threonine activity has been implicated, or is suspected in a number of pathologies including septic shock, bone loss, psoriasis, rheumatoid arthritis, many cancers and other proliferative diseases (See, U.S. Patent No. 6,165,716 to Creasy et al. (Issue Date: Dec. 20, 2000)). A number of researchers have expended considerable time to identify serine/threonine protein kinases that may play a role in preventing, ameliorating and correcting dysfunctions or diseases. For example, U.S. Patent No. 5,972,606 to Creasy et al. (Issue Date: October 26, 1999), discloses a human protein serine/threonine kinase, designated HOACF72, of the hYAKl family of polypeptides, antibodies against which are said to be useful in the treatment of bone loss, inflammatory diseases such as rheumatoid arthritis, osteoarthritis, adult respiratory disease syndrome (ARDS), inflammatory bowel disease (IBD), psoriasis, dermatitis, asthma, allergies, infections, septic shock, pain, cancers, anorexia, bulimia, and a host of other conditions. U.S. Patent Nos. 5,965,420 and 6,165,766, also to Creasy et al. (Issue Dates: October 12, 1999 and December 26, 2000, respectively), assert human YAK3 polypeptides and polynucleotides, antibodies against which are said to be useful for treating bone loss, inflammatory diseases, infections, immunodeficiency disorders, septic shock, pain, cancers and a host of other pathological conditions. As stated by Creasy *et al.,* there is a need for further identification and characterization of further members of the serine/threonine protein kinase family to identify other members of the family that may play a role in preventing, ameliorating or correcting dysfunctions or diseases. There is also a need to identify potential relationships between these kinases and disease states themselves.

### SUMMARY OF THE INVENTION

The prior art has emphasized the role of *Pim-2* in oncogenic behavior, and has classified the gene as a proto-oncogene. It was particularly surprising that the present inventors have found that transcription of *Pim-2* is significantly increased in a variety of inflammatory states, with particularly large increases in *Pim-2* mRNA seen with respect to intestinal tissue levels in patients diagnosed with ulcerative colitis and Crohn's disease and inflammatory disease states associated with an inflamed: pancreas, tonsils, bowel (including small and large intestines and rectum), stomach lining, thyroid, cervix, lung, kidney, liver, and skin.

The present invention relates to polynucleotide sequences encoding human Pim-2 (h-*Pim-2*) and h-Pim-2 polypeptides. One embodiment of the invention relates to methods for using such polynucleotides and polypeptides for the treatment of human inflammatory diseases, such as ulcerative colitis and Crohn's Disease. Another embodiment of the invention relates to methods for screening compounds for potential anti-inflammatory activity by adjudging the effect of such compounds on Pim-2 activity. And yet another embodiment of the present invention relates to diagnostic assays for detecting inflammatory diseases associated with altered Pim-2 activity.

In one embodiment of the present invention, there is disclosed a method for diagnosing inflammatory disease states, such as an inflammatory bowel disease, using a tissue sample obtained from a patient, said method comprising the steps of: (a) measuring the level of Pim-2, or Pim-2 mRNA, in the tissue sample of the patient; and (b) determining any difference of the level of Pim-2 or Pim-2 mRNA in the tissue sample of the patient as compared to the level of Pim-2 or Pim-2 mRNA in comparable tissue sample(s) obtained from one or more patients lacking the inflammatory disease state. Such method may further comprise the step of (c) diagnosing the patient as having the inflammatory disease state when the measurement of such parameter with respect to the patient's tissue is significantly higher than in comparable tissue sample(s) obtained from the one or more patients lacking the inflammatory disease state. By "significantly higher" it is meant a difference of more than about 50%, more preferably more than about 100%, and yet more preferably more than about 200%. The level of Pim-2 or Pim-2 mRNA may be measured directly, or indirectly, as for example by measuring kinase activity of Pim-2. Such method may comprise *in situ* hybridization of at least one nucleic acid probe comprising a polynucleotide sequence of at least about 15 contiguous nucleotides of SEQ ID NO: 1, preferably a nucleic acid probe includes nucleotides 294 through 311 of SEQ ID NO:1. Such method may be particularly advantageously used to diagnosis Crohn's Disease and ulcerative colitis, but may also be used to detect inflammatory disease states associated with an inflamed pancreas, tonsils, bowel (including small and large intestines and rectum), stomach lining, thyroid, cervix, lung, kidney, liver, and skin.

In another embodiment of the present invention, there is provided a method for diagnosing an inflammatory disease state, such as an inflammatory bowel disease, in a patient comprising the steps of (a) establishing a statistically significant correlation between Pim-2 expression in the inflamed tissue of the inflammatory disease state, and the presence and/or severity of the inflammatory disease state; (b) measuring the Pim-2 level in corresponding tissue obtained from said patient; and (c) determining whether the measured Pim-2 level corresponds to a level correlated with the inflammatory disease state. Such method may also be particularly advantageously used to diagnosis Crohn's Disease and ulcerative colitis, but may also be used to detect inflammatory disease states associated with an inflamed pancreas, tonsils, bowel (including small and large intestines and rectum), stomach lining, thyroid, cervix, lung, kidney, liver, and skin.

There is also provided a method for monitoring the efficacy of anti-inflammatory drug regimens in the treatment of an inflammatory disease state, such as an inflammatory bowel disease, said method comprising the steps of: (a) establishing a statistically significant correlation between Pim-2 levels and clinical response to anti-inflammatory therapy in the inflammatory disease state; (b) measuring the Pim-2 level in the patient; and (c) determining the correspondence between the Pim-2 level measured in the patient and the Pim-2 levels correlated to clinical response to anti-inflammatory therapy. This method may advantageously be employed to monitor the efficacy of anti-inflammatory drug regimens with respect to the treatment Crohn's Disease and ulcerative colitis. This method may also be employed to monitor the efficacy of anti-inflammatory drug regimens with respect to inflammatory disease states associated with an inflamed pancreas, tonsils, bowel (including small and large intestines and rectum), stomach lining, thyroid, cervix, lung, kidney, liver, and skin.

Other methods for detecting inflammatory disease states, such as an inflammatory bowel disease, are also encompassed by the present invention. For example, the present invention further provides a method for detecting inflammatory disease states comprising the steps of: (a) collecting a suspect sample, and (b) subjecting the suspect sample to a diagnostic test employing the nucleotide sequence of SEQ ID NO:1, or fragments thereof, the diagnostic test comprising polymerase chain reaction or nucleic acid hybridization or (a) collecting a suspect sample, and (b) subjecting the sample to a diagnostic test comprising polyclonal antisera and/or monoclonal antibody raised to immunogens comprising the polypeptide sequence of SEQ ID NO:2, or immunogenic fragment thereof, said diagnostic test comprising Western blot analysis or enzyme-linked immunoassay (ELISA). It also provides a method for detecting inflammatory disease states comprising the steps of: (a) collecting a suspect sample, and subjecting the sample to a diagnostic test comprising polyclonal antisera and/or monoclonal antibody raised to immunogens comprising the polypeptide sequence of SEQ ID NO:2, or immunogenic fragment thereof, (b) detecting said polyclonal antisera and/or monoclonal antibody.

A diagnostic kit for detecting inflammatory disease states is also encompassed by the present invention. Such diagnostic kit may comprise, for example: (a) an antibody specific for SEQ ID NO:2 or an antigen-binding portion of an antibody specific for SEQ ID NO:2; and (b) reactants for detecting said antibody or portion specific for SEQ ID NO:2.

The present invention also provides for screening assays for determining whether a compound would be effective in the treatment of an inflammatory disease state. One such screening assay comprises the steps of: (a) incubating the compound with cells that express SEQ ID NO:2, or variant thereof, upon exposure to LPS; (b) determining the extent of inhibition caused by said compound on the expression of SEQ ID NO:2, or variant thereof, by measuring a parameter indicative of the level of SEQ ID NO:2 (or variant thereof) or m-RNA translated to SEQ ID NO:2 (or variant thereof). Another such screening assay comprises: (a) incubating *in vitro* the compound with a protein comprising SEQ ID NO:2, or variant thereof, having kinase activity, and a substrate with respect to said kinase activity; (b) determining whether the compound inhibits the kinase activity of the protein with respect to the substrate. The protein of this assay may be of recombinant or natural origin. Compounds identified by such screening assays are also encompassed by the present invention.

Another screening assay for identifying compounds that ameliorate inflammatory disease states comprises the steps of: (a) separately cultivating a first immortalized cell line containing at least one gene of SEQ ID NO:1, and a second immortalized cell line wherein the gene of SEQ ID NO: is inactivated; (b) subjecting both cell lines to a compound suspected of having anti-inflammatory activity; and (c) determining if said compound selectively inhibits growth of said first immortalized cell line. Again, compounds identified by such assay are within the scope of the present invention.

And yet in another embodiment of the present invention, there is provided a screening assay (and compounds identified thereby) for identifying compounds that may have use in the amelioration of inflammatory disease states, such as an inflammatory bowel disease, due to modulation or alteration of Pim-2 activity, comprising the steps of: (a) establishing a control system comprising Pim-2 and a substrate of Pim-2; (b) establishing a test system comprising Pim-2, said substrate of Pim-2 and the candidate compound; (c) measuring the activity of Pim-2 in the control and test systems; and (d) determining that the candidate compound modulates or alters Pim-2 activity if the activity of Pim-2 in the test system is less than or greater than the activity measured for the control system. The screening assay may also comprise contacting a compound with a cultured cell that expresses the *Pim-2* gene, and detecting a change in the expression of the *Pim-2,* or kinase activity of Pim-2, in the cultured cell. This method may further comprise the step of- determining that a screened compound is useful in the treatment of inflammatory disease states when the expression of the *Pim-2* gene, or kinase activity of Pim-2, in the cultured cell is significantly diminished by the screened compound. By "significantly diminished" it is meant that the expression of the *Pim-2* gene, or kinase activity of Pim-2, is reduced by more than about 50%, more preferably 100%, and yet more preferably 200%. These methods may also be employed for identifying compounds that may have use in the amelioration of inflammatory disease states associated with an inflamed pancreas, tonsils, bowel (including small and large intestines and rectum), stomach lining, thyroid, cervix, lung, kidney, liver, and skin.

Alternatively, compounds can be screened for activity in the treatment of inflammatory disease states and inflammatory disease states associated with an inflamed pancreas, tonsils, bowel (including small and large intestines and rectum), stomach lining, thyroid, cervix, lung, kidney, liver, and skin by measuring the affinity of the compounds for Pim-2.

In the screening methods of the present invention, a change in the expression level of pro-inflammatory cytokines, such as IL-6, compared to control is an indication of Pim-2 activity. Differences in expression levels may be determined using methods known in the art including but not limited to RNA interference (RNAi) technology (Elbashir, S.M. et al, 2001, Nature, 411, 494-498).

Candidate compounds identified and/or isolated by any of these methods are also encompassed by the present invention.

Methods for treating animals inflicted with inflammatory disease states, and preventing the development of inflammatory disease states, including but not limited to an inflammatory bowel disease, are also disclosed.

In one method, treatment is accomplished by administration of a therapeutically or prophylactically effective amount of an antisense compound targeted to a nucleic acid sequence encoding *Pim-2.* In yet another embodiment, there is provided a method for treating an inflammatory disease state, such as an inflammatory bowel disease, which comprises administering to a patient in need thereof an oligonucleotide which specifically hybridizes to a transcript encoding human Pim-2 and suppresses the expression of the human Pim-2, as its effective ingredient, and a pharmacologically acceptable carrier. In another method, there is administered to a patient an amount of an agent that inhibits Pim-2 production, wherein the agent is an antisense construct that targets Pim-2 encoding sequences, under conditions that the treatment is effected. In another method, the agent is a short interfering (si) RNA construct that targets *Pim-2* encoding sequences.

The above methods of diagnosing, monitoring efficacy of antiinflammatory drugs, detecting, screening, and identifying work particularly well with respect to inflammatory bowel diseases, in particular Crohn's Disease and Ulcerative Colitis.

### BRIEF DESCRIPTION OF THE DRAWINGS

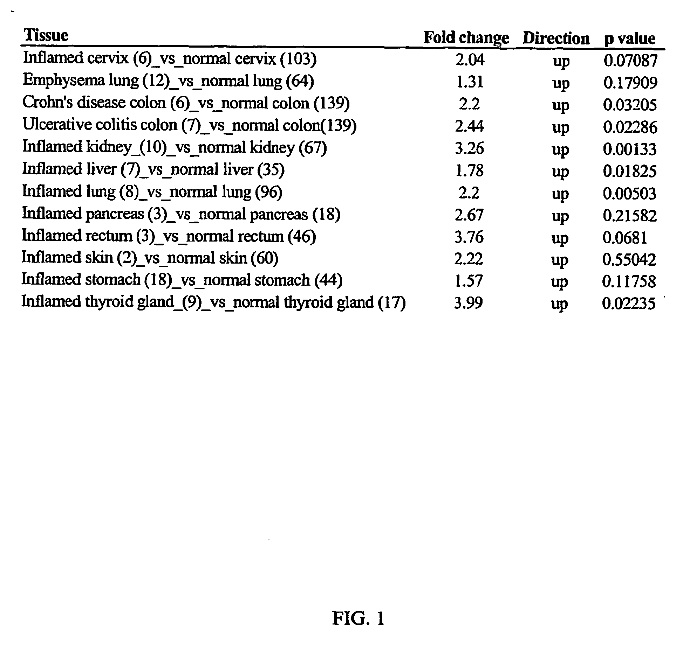

*FIG. 1* shows the fold changes of *Pim-2* mRNA expression in persons suffering different inflammatory disease states as compared to *Pim-2* mRNA expression in persons lacking such inflammatory disease states. The number of donor samples tested is indicated by (n). mRNA expression levels were obtained by Affymetrix Gene Chip arrays as described (Lockhart, D.J. et al., Nat. Biotechnol. 14: 1675-1680 (1996)). Confidence p-values were calculated based on a two-sided Welch modified two-sample t-test.

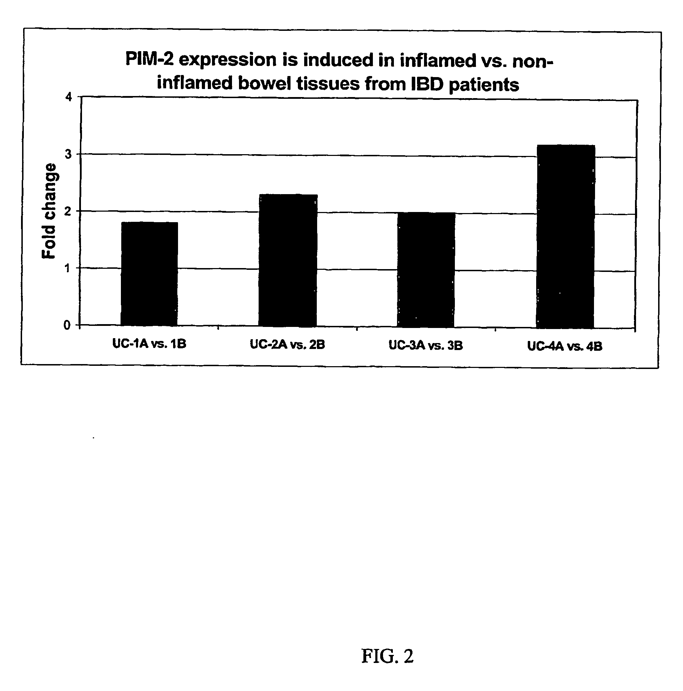

*FIG. 2* shows *Pim-2* m-RNA expression in inflamed bowel tissue as compared to *Pim-2* mRNA expression in non-inflamed bowel tissue of the same patients diagnosed with ulcerative colitis. "A" indicates "inflamed" and "B" indicates "non-inflamed" tissue. The four different patients are numbered as 1, 2, 3, and 4.

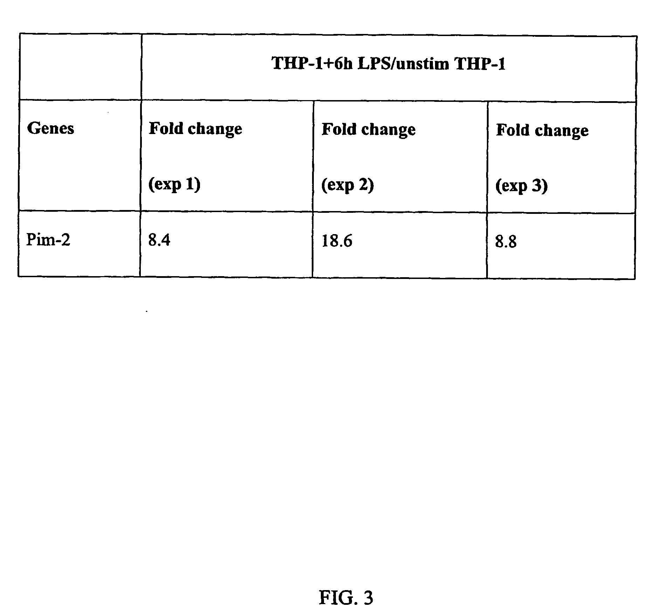

*FIG. 3* shows the results of three experiments wherein *Pim-2* expression was measured in THP-1 cell lines stimulated and unstimulated with lipopolysaccharide. Fold change values were derived from the comparison of *Pim*-2 mRNA expression in THP-1 cells stimulated with LPS for 6 hours versus Pim-2 mRNA expression in unstimulated THP-1 cells.

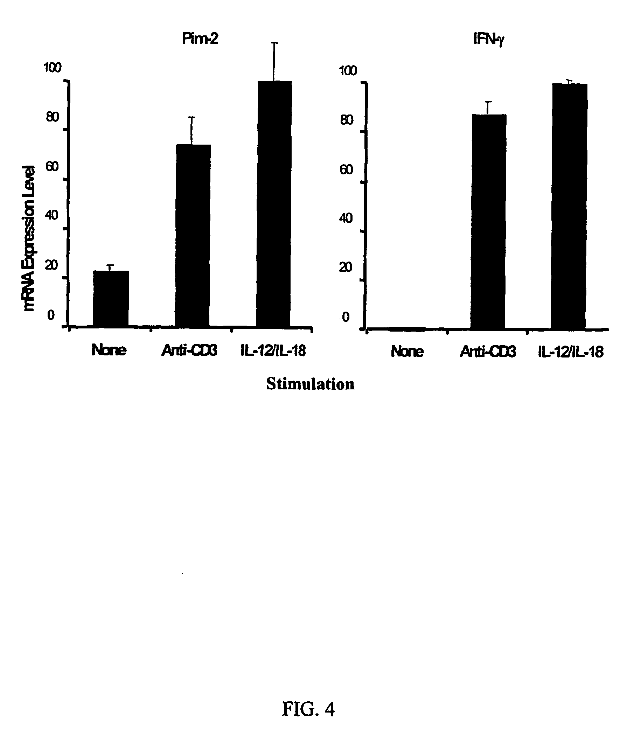

*FIG. 4* shows *Pim*-2 mRNA is induced by anti-CD3 or IL-12/IL-18 stimulation in CD4+ Th1 cells. DO11.10 splenic cells were stimulated with OVA, IL-12 and anti-IL-4 for 7 days. CD4+ cells were harvested and stimulated with anti-CD3 or IL-12/IL-18 for 16 hours; the total RNA was extracted and first strand cDNA was synthesized. The mRNA of *Pim*-2 and IFN-γ were detected by TaqMan analysis. The mRNA expression levels of each gene are presented as percentage values of the mean mRNA copy number in IL-12/IL-18 stimulated sample.

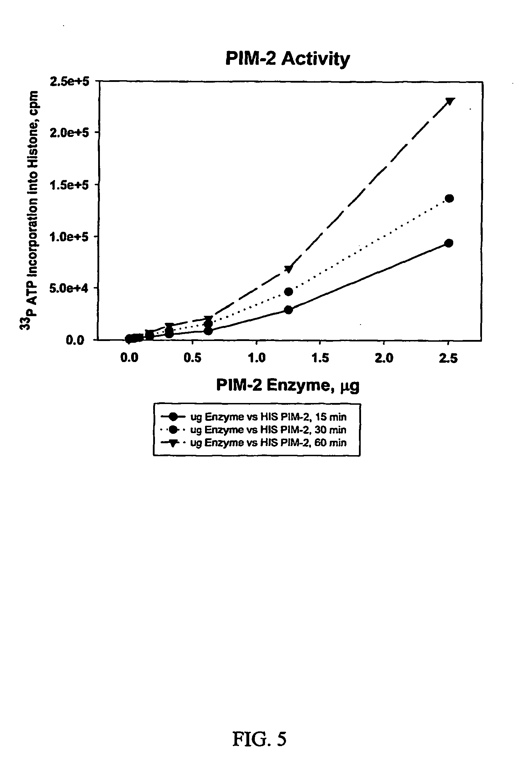

*FIG. 5* shows that purified recombinant Pim-2 is active in phosphorylating Histone. His-tagged *Pim*-2 was expressed and purified from *E. coli.* Indicated amounts of Pim-2 were assayed in a buffer containing 25 mM HEPES pH 7.5, 10 mM MgCl₂, 0.5 mM DTT, 10 µM cold ATP, 1.5 µCi [γ-³³P]-ATP, 10 µg Histone III (type ss from calf thymus) at room temperature (-●- µg enzyme vs. His- *Pim*-2, 15 min.; ··●·· µg enzyme vs. His- *Pim*-2, 30 min.; -▼- µg enzyme vs. His- *Pim*-2, 60 min.). Incorporation of ³³P into Histone was measured.

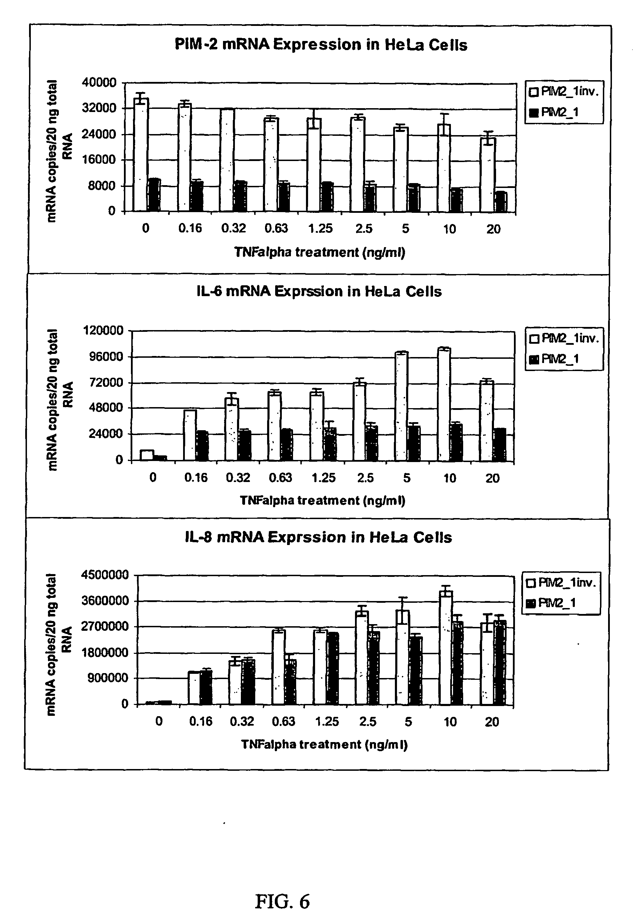

*FIG. 6* shows that Pim-2 is required for TNF-α-induced IL-6 expression in HeLa cells. *Pim*-2 siRNA duplexes (PIM2_1 inverted control and PIM2_1at 200nM) were transfected into HeLa cells for 2 days. Then, the cells were treated with various concentrations (between 0.16 and 20 ng/ml) of TNF-α for 2 hours before their total cellular RNA was prepared for TaqMan real-time PCR analysis. RT and PCR were carried out using TaqMan quantitation (showing mRNA copy numbers detected in 20 ng total RNA). The copy numbers of gene transcripts were determined according to DNA standards and normalized with human *Gapdh.* All TaqMan PCR reactions of each individual sample were performed in triplicate, then the copy numbers and standard error were determined.

### DETAILED DESCRIPTION OF THE INVENTION

### 1. Definitions

The following definitions are provided to facilitate understanding of certain terms used herein:
By "antibodies" it is meant to include polyclonal and monoclonal antibodies, chimeric, single chain, and humanized antibodies, as well as Fab fragments, including the product of an Fab or other immunoglobulin expression library.
By "cells" it is meant to include cells in any form, including, but not limited to, cells retained in tissue, cell clusters and individually isolated cells.
By "cell line" it is meant a clone of a primary cell that is capable of stable growth *in vitro* for many generations.
By "clone" it is meant a population of cells derived from a single cell or common ancestor by mitosis.
By a DNA "coding sequence" it is meant a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxy) terminus. A polyadenylation signal and transcription termination sequence will usually be located 3' (downstream) to the coding sequence.
By "exogenous" material it is meant material that has been introduced into a cell, organism etc. that originated outside of the same.
By "heterologous" region of a DNA construct it is meant an identifiable segment of DNA within a larger DNA molecule that is not found in association with the larger molecule in nature.
By "isolate" a material it is meant changing the environment of the material or removing a material from its original environment, or both. For example, when a polynucleotide or polypeptide is separated from the coexisting materials of its natural state, it is "isolated."
By "operably linked" nucleotide sequences it is meant a juxtaposition such that the functionality of the sequences is preserved. Thus, for example, a coding sequence "operably linked" to a promoter is positioned so that the promoter is capable of effecting the expression of the coding sequence.
By "polynucleotide" it is meant any polyribonucleotide or polydeoxyribonucleotide, which may be unmodified RNA or DNA, or modified DNA or DNA. As used herein, "polynucleotide" include, without limitation, single- and double-stranded DNA and RNA, hybrid molecules comprising DNA and RNA that may be single-stranded, or more typically double-stranded, or a mixture of single- and double-stranded regions. The term "polynucleotide" further may refer to triple-stranded regions comprising RNA or DNA or both DNA and RNA. "Polynucleotide" embraces chemically, enzymatically or metabolically modified forms of polynucleotides typically found in nature, as well as chemical forms of DNA and RNA characteristic of viruses and cells. The term is meant to encompass both long nucleotide as well as short nucleotide sequences, often referred to as oligonucleotides, and oligomers.
By the term "polypeptide" it meant to refer to any peptide or protein comprising two or more amino acids joined to each other by peptide bonds or modified peptide bonds, i.e. peptide isosteres. Polypeptides may comprise amino acids other than the 20 gene-encoded amino acids, and includes amino acids modified either naturally or synthetically.
By "Pim-2 polypeptide" it is meant to include SEQ ID NO:2, and polypeptides comprising an amino acid sequence of SEQ ID NO:2 that have at least 80% identity, still more preferably 90% identity, and still more preferably 95% identity, with the sequence of SEQ ID NO:2 over its entire length. The Pim-2 polypeptide may be in the form of the "mature" protein or may be a part of a larger protein such as a fusion protein, and may include secretory or leader sequences, pro-sequences, sequences which aid in purification, or additional sequence for stability during recombinant production.
By "recombinant" or "engineered" cell it is meant a cell into which a recombinant gene has been introduced through the hand of man. Recombinantly introduced genes may be in the form of a cDNA gene (i.e., lacking introns), a copy of a genomic gene (i.e., including introns with the exons), genes produced by synthetic means, and/or may include genes positioned adjacent to a promoter, or operably linked thereto, not naturally associated with the particular introduced gene.
By "replicon" it is meant any genetic element (*e.g.,* plasmid, chromosome, virus) that functions as an autonomous unit of DNA replication *in vivo*, i.e. capable of replication under its own control.
By "transformed cell" it is meant a cell into which exogenous or heterologous DNA has been introduced. The transforming DNA may or may not be integrated (covalently linked) into chromosomal DNA making up the genome of the cell. The transforming DNA may be maintained on an episomal element such as a plasmid.
By "variant" it is meant a sequence, such as a polynucleotide or polypeptide, that differs from another sequence, but retains essential properties. For example, a variant of a polynucleotide may differ in nucleotide sequence by one or more substitutions, additions, and deletions, from the reference polynucleotide.
By "vector" it is meant a replicon, such as a plasmid, phage or cosmid, to which another DNA segment may be attached so as to bring about the replication of the attached segment.

### 2. Diagnostic Assays for the Determination of Inflammatory Disease States

The present inventors have surprisingly discovered that *Pim-2* transcription and translation is significantly enhanced in inflammatory disease states. In particular, as shown in *Fig. 1**,* the present inventors have discovered that expression of h-Pim-2, and its m-RNA template, are dramatically increased in select tissues of humans diagnosed with ulcerative colitis and Crohn's disease, two inflammatory bowel diseases, inflamed thyroid diseases, inflamed stomach diseases, inflamed pancrease diseases, inflamed cervix, inflamed lung tissue, inflamed kidney, inflamed liver, and inflamed skin, as compared to tissue of humans without such inflammatory disease states or in remission from such inflammatory disease states (controls). Increases in expression of h-Pim-2, and its m-RNA template, have also been noted in tonsillitis, thyroiditis and inflamed rectal disease. As seen in *Fig.* 2, mRNA expression of h-*Pim-2* was significantly higher (2-3 fold) in inflamed colon tissues of persons suffering from the inflamed bowel disease ulcerative colitis as compared to non-inflamed colon tissue.

Inflammatory diseases may be diagnosed by methods comprising determining from a sample derived from a subject the extent of transcription and translation of *Pim-2* as compared to transcription and translation of the gene in a normal population (i.e., not suffering from the inflammatory disease). Characterization of expression at the RNA level may be made using any of the methods well known in the art for the quantization of polynucleotides, such as, for example, PCR, RT-PCR, RNase protection, Northern blotting and other hybridization methods. Levels of Pim-2 polypeptide can be assayed likewise using techniques well known to one of ordinary skill in the art, such techniques including, but not limited to, competitive-binding assays, Western Blot analysis and ELISA assays. Microarray technology is well known and has general applicability to gauge gene expression.

### 3. Screening Assays

The unexpected discovery that *Pim-2* expression is significantly enhanced at both the transcriptional and translational level in inflammatory disease states proffers new screening procedures to isolate compounds that diminish (or increase) the severity of the inflammatory disease state.

Agonists or antagonists of Pim-2, or of the transcription and/or translation of the *Pim-2* gene, may find use in the treatment of inflammatory states.

Antagonists may be employed for therapeutic and prophylactic purposes to decrease inflammation by decreasing Pim-2 activity in the affected tissue or organ. Antagonists of Pim-2 activity may find particular use in ameliorating inflammatory bowel diseases, inflammatory thyroid diseases, inflammatory stomach diseases, and inflammatory pancreas diseases wherein increased expression of the gene is seen to be high in affected individuals. Antagonists may also find use in ameliorating inflammatory conditions seen in other tissues, including, but not limited to, the lung, skin, kidney , and thyroid. As Pim-2 is expressed widely in tissues of the body, antagonists of Pim-2 baseline activity may find use in a variety of inflammatory states other than these inflammatory diseases, including (but not limited to) adult respiratory disease syndrome (ARDS), allergies, asthma, dermatitis, osteoarthritis, psoriasis, rheumatoid arthritis.

Screening procedures may entail utilization of appropriate cells that express Pim-2 or respond to Pim-2 polypeptide of the present invention. Such cells include cells from mammals, yeast, *Drosophila* or *E. coli.* One particularly useful cell-line is THP-1, a human acute monocytic leukemia cell line available from American Type Culture Collection, Rockville, MD (USA) which displays lymphoblastic-like cell morphology, has Fc and C3b receptors and lack surface and cytoplasmic immunoglobulins (these cells stain positive for alfa-naphthyl butyrate esterase, produce lysozymes and are phagocytic). Cells that express Pim-2, or respond to Pim-2, may be contacted with a test compound to determine the effect of the test compound on Pim-2 activity. Test compounds demonstrating action to reduce Pim-2 activity with respect to such cells may be considered good candidates as therapeutic agents in treating inflammatory disease states.

Pim-2 expressing cells may be cells transformed so as to express SEQ ID NO:2, or a variant thereof. The Pim-2 polypeptide of SEQ ID NO:2 may be prepared in both prokaryotic and eukaryotic systems. Constructs may be made wherein the coding sequence for the polypeptide is preceded by an operable signal peptide which results in secretion of the protein. The particulars for construction of expression systems and purification of peptides, and cleavage from fusion peptides are well known to those of ordinary skill in the art. Technology for introduction of DNA into cells includes four general methods: (1) physical methods such as microinjection, electroporation and the gene gun (See, *eg.,* Johnston et al., Gene gun transfection of animal cells and genetic immunization, 43(A) Methods Cell. Biol. 353 - 365 (1994)); (2) viral vectors (See, *e.g.,* Eglitis et al., Retroviral vectors for introduction of genes into mammalian cells, 6(7) Biotechniques 608 - 614 (1988)); (3) chemical methods (See, *e.g.,* Zatloukal et al., Transferrinfection: A highly efficient way to express gene constructs in eukaryotic cells, 660 Ann. N.Y. Acad. Sci. 136 - 153 (1992)), and (4) receptor-mediated mechanisms (See, *e.g.,* Wagner et al., Coupling of adenovirus to transfetrin-polylysine/DNA complexes greatly enhances receptor mediated gene delivery and expression of transfected genes, 89(13) Proc. Natl. Acad. Sci. USA 6099 - 6103 (1992)).

As would be understood by one of ordinary skill in the art, minor modification of the primary amino acid sequence of SEQ ID NO:2 may result in a polypeptide that has substantially equivalent activity as compared to SEQ ID NO:2. By "modification" of the primary amino acid sequence it is meant to include "deletions" (that is, polypeptides in which one or more amino acid residues are absent), "additions" (that is, a polypeptide which has one or more additional amino acid residues as compared to the specified polypeptide), "substitutions" (that is, a polypeptide which results from the replacement of one or more amino acid residues), and "fragments" (that is, a polypeptide consisting of a primary amino acid sequence which is identical to a portion of the primary sequence of the specified polypeptide). By "modification" it is also meant to include polypeptides that are altered as a result of post-translational events which change, for example, the glycosylation, amidation, lipidation pattern, or the primary, secondary, or tertiary structure of the polypeptide.

It is known in the art that certain amino acids may be substituted by other amino acids having a similar hydropathic index or score and still result in a polypeptide having similar biological activity. In making such changes, the substitution of amino acids whose hydropathic indices are within ±2 is preferred, those that are within ±1 are more preferred, and those within ±0.5 are even more preferred. Similarly, select amino acids may be substituted by other amino acids having a similar hydrophilicity, as set forth in U.S. Pat. No. 4,554,101 (herein incorporated by reference in its entirety). In making such changes, as with the hydropathic indices, the substitution of amino acids whose hydrophilicity indices are within ±2 is preferred, those that are within ±1 are more preferred, and those within ±0.5 are even more preferred (See, *e.g.,* Kyte et al., 157 J. Mol. Biol. 105 - 132 (1982), herein incorporated by reference in its entirety).

Conservative amino acid changes may be achieved by changing the codons of the DNA sequence using for example known redundancy in the code:

**TABLE 1**

| **Amino Acid** | **Three-Letter Designation** | **Single Letter Designation** | **Codons** |
|---|---|---|---|
| Alanine | Ala | A | GCA GCC GCG GCU |
| Cysteine | Cys | C | UGC UGU |
| Aspartic Acid | Asp | D | GAC GAU |
| Glutamic Acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUG CUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGG CGU |
| Serine | Ser | S | AGC AGU UCA UCC UCG UCU |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |

In this aspect, the present invention also relates to vectors which comprise Pim-2, or variant thereof, and host cells which are genetically engineered with vectors of the invention, and to the production of Pim-2 polypeptides by recombinant techniques. Cell-free translation systems may also be employed to produce such proteins using RNAs derived from the DNA constructs of the present invention.

The *Pim-2* polynucleotide of SEQ ID NO: 1 may be obtained using standard cloning and screening, for example, from a cDNA library derived from mRNA or from genomic DNA libraries, or may be synthesized using well known and commercially available techniques. When the polynucleotide is used for recombinant production, that is to produce recombinant cells, it may consist of the mature polypeptide, or fragment thereof, of may include other coding sequences such as a leader or secretory sequence, a pre-, or pro- or pre-pro sequence, or other fusion peptide portions.

Host cells that are to be transformed may be genetically engineered to incorporate expression systems of the Pim-2 polypeptide. Introduction of the *Pim-2* expression polynucleotide sequence into the host cell can be effectuated by any of the methods well known to those of ordinary skill in the art as described, for example, in Sambrook et al., Molecular Cloning: A Laboratory Manual, 2d Ed., Cold Spring Harbor Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection. Generally, any system or vector suitable to maintain, propagate or express the polynucleotide to produce the polypeptide may be used.

The present inventors have further demonstrated that the *Pim-2* gene is a bona fide NF-κB target by virtue to its response to a transdominant IκBαSR (super repressor), and that its expression may be induced by lipopolysaccharide ("LPS") (J. Biol. Chem. 276: 13579 (2001)). Studies performed by the present inventors suggest that up-regulation of *Pim-2* in cells by LPS is controlled by the HCK/NF-κB pathway. The NF-κB signal transduction pathway involves a series of intracellular steps that promote phosphorylation and subsequent dissociation of IκB inhibitor protein from the inactive NF-κB complex. It is believed that liberated NF-κB translocates to the nucleus where it binds to the k enhancer element on the DNA and may activate transcription of *Pim-*2 gene. As the NF-κB signal transduction pathway is also induced by TNF, IL-1 and phorbol ester, these compounds as well may be used to induce Pim-2 activity.

Expression with LPS of various cell lines, including human monocytes (THP-1) and mouse pre-B cells, has been seen by the present inventors to increase about 10 fold. An LPS (or other inducer of the NF-κB signal transduction pathway) -stimulated cell line may thus be used advantageously to improve the detection of compounds which may possess anti-inflammatory activity associated with decreased Pim-2 base line activity. *Fig. 3* illustrates three different experiments undertaken to determine the fold change of Pim-2 RNA transcription in THP-1 cell lines, the fold change ranging from 8.4 to 18.6.

Crohn's Disease is mediated, *inter alia,* by activated Th1 cells. T cell cultures from patients with Crohn's Disease produce significantly higher levels of IFN-γ and TNF-α than T cell cultures from healthy controls (Agnholt, J. and K. Kaltoft, Cytokine 15(4):212-222 (2001)). *Fig. 4* illustrates an experiment undertaken to determine induction of *Pim-2* and IFN-γ by anti-CD3 or IL-12/IL-18 stimulation of CD4⁺ Th1 cells. As can be seen in *Fig. 4*, *Pim-2* mRNA expression in stimulated CD4⁺ Th1 cells was significantly increased compared to unstimulated control cells.

Cell lines may alternatively, or may also, be activated to express Pim-2 by exposing the cells to Moloney murine leukemia proviruses.

Screening procedures may also entail a test to determine the binding of a candidate compound with Pim-2 itself. Binding may be detected by any of the methods well known in the art, as by means of a label directly or indirectly associated with a candidate compound or by measuring competition with a labeled competitor, such as an agonist of Pim-2 activity identified by the present invention which is found to bind to Pim-2 itself. Standard methods for conducting such screening assays are well understood in the art. Indicators of Pim-2 activity may include but are not limited to differences in kinase activity compared to control or changes in expression levels of pro-inflammatory substances, for example, TNF-α, IL-6, and IFN-γ.

### 4. Treatment of Inflammatory States Employing Agents Directed to Pim-2 Gene and Polypeptide

The polynucleotide sequence of Pim-2 is reported at GenBank Accession Nos. NM_006875/U77735 based on sequence data reported by Baytel et al., Biochim. Biophys. Acta 1442: 274 (1998) (SEQ ID NO:3). The present inventors have discovered that the reported polynucleotide coding sequence of Pim-2 by Baytel *et al.* differs from that obtained by them in the sequencing of image EST clones comprising the *Pim-2* gene. The Baytel *et al.* polynucleotide Pim-2 sequence indicates an additional thymidine ("t") at position 1063 or 1064 in the coding region as opposed to SEQ ID NO: 1, obtained by the present inventors, and a coding sequence at positions 185 - 1120, as opposed to a coding sequence 185 to 1117 obtained by the present inventors.

As is well known in the art, a single insertion (or deletion) in a nucleotide sequence compared to the actual sequence will cause a frame shift in translation of the nucleotide sequence such that the predicted amino acid sequence encoded by a determined nucleotide sequence will be different from the amino acid sequence actually encoded by the sequenced DNA molecule, beginning at the point of such an insertion (or deletion). The present inventors have determined that such is the case with respect to the amino acid sequence reported by Baytel *et al.* The Baytel *et al.* sequence (SEQ ID NO:4) indicates that Pim-2 has 41 amino acids not found in SEQ ID NO:2 and that it comprises twenty-three additional amino acids as compared to that SEQ ID NO:2 due to a belated stop codon. The present inventors have determined that SEQ ID NO: 1 encodes wild-type Pim-2. The Baytel *et al.* polynucleotide sequence for *Pim-2* may differ from wild-type due to the source material for its sequencing comprising a polymorphism in the form of an addition mutation, or may have resulted from a sequencing error.

Support for polypeptide SEQ ID NO:2 is found in genomics sequence data supplied by Ishida *et al.* at NCBI:AB042425 with respect to the genomic organization of human UDP-galactose transporter gene from which they predict a *Pim-2* proto-oncogene "homolog" polypeptide (SEQ ID NO:5) that corresponds to the *Pim-2* polypeptide uncovered by the present inventors. To date, there has been a lack of consistency in predicting human genes from genomic sequences (Hogenesch, et al., Cell 106: 413-415 (2001)). Because of potential errors of exons predicted from genomic sequences, Ishida *et al.* could not point out the mutation of sequence error in the cDNA record of h-*Pim-2* (accession:U77735), instead, they named the predicted peptide sequence as a *Pim-2* homologue. Support for polynucleotide SEQ ID NO: 1, as well as polypeptide SEQ ID NO:2, is found at NCBI:XM_010208 (SEQ ID NO:6) and NCBI:XP_010208 (SEQ ID NO:7), both directly submitted by the National Center for Biotechnology, which based on sequence data derived by automated computational analysis of NCBI genomic sequence contig NT_011611, using Assembly gene prediction methodology, indicate polynucleotide and polypeptide sequences corresponding to those found by the present inventors.

*Fig. 5* illustrates that Pim-2 polypeptide as characterized by the present inventors is capable of phosphorylating histones in a concentration dependent manner.

The invention contemplates the amelioration and/or treatment of such diseases by administering a Pim-2 inhibiting amount of an inhibitor of Pim-2 activity. As would be understood by one of ordinary skill in the art, useful inhibitors of Pim-2 activity at the cellular level include, but are not limited to, compounds that inhibit the kinase activity of Pim-2, and/or reduce the expression of Pim-2 at either the transcription or translation level, and/or increase the degradation of Pim-2, and/or inhibit the interaction of Pim-2 with one or more of its upstream or downstream modulators/substrates, and include antibodies, or fragments or analogues thereof. In one embodiment of the present invention, the inhibitor of Pim-2 activity is identified by means of the screening test described above.

Inflammatory disease states may be treated by inhibiting the expression of the *Pim-2* gene using expression blocking techniques, such techniques being known to those of ordinary skill in the art. For example, such techniques may involve the use of antisense sequences, either internally generated or separately administered (See, *e.g.*, O'Connor, J. Neurochem. 56: 560 (1991) or the formation of triple helices with the gene (See, *e.g.,* Dervan et al., Science 251:1360 (1991)). Alternatively, such techniques may utilize RNA interference (RNAi) technology (also referred to as short interfering RNA (siRNA) technology). *Fig.* 6 illustrates gene knock-down studies of *Pim-2* in HeLa cells using the siRNA technology (Elbashir, S.M. et al, 2001, Nature, 411, 494-498). SiRNA oligo (Pim-2-1; sense: 5'-GUGAUUCCCCGGAAUCGUGTT-3' (SEQ ID NO:8), antisense: 5'-CACGAUUCCGGGGAAUCACTT-3' (SEQ ID NO:9) was designed to specifically knock down mRNA expression of *Pim-2.* The inverted siRNA oligo (Pim2-1 inv; sense: 5'-GUGCUAAGGCCCCUUAGUGTT-3' (SEQ ID NO:10), antisense: 5'-CACUAAGGGGCCUUAGCACTT-3' (SEQ ID NO:11)) was used as a control. The role of Pim-2 in mediating inflammation can at least be explained by its function in controlling the expression of interleukin-6 (IL-6). IL-6 is a major pro-inflammatory cytokine. Increased production of IL-6 has been reported in both Crohn's Disease and ulcerative colitis disease (Braegger CP et al., 1994, Ann Allergy, 72,135-141). IL-6-/- mice display defective inflammatory response (Fattori E et al., J Exp Med 1994, 180:1243-1250). As shown in *Fig.* 6, when *Pim-2* expression was suppressed by its siRNA oligo (Pim-2-1), the expression of IL-6 was reduced when cells were stimulated with various doses of TNF-α. Such repression is gene-specific, since the same siRNA had no significant effect on IL-8 production in response to TNF-α (Fig. 6).

Inflammatory disease states may also be treated by means of antibodies, or vaccines formulated to induce an immunological response in the affected animal so as to interfere with Pim-2 activity in the cell. For example, antibodies may be generated against the Pim-2 polypeptide of the present invention by administering Pim-2, or an epitope-bearing fragment thereof, to an animal capable of generating such antibodies using routine protocols. For preparation of monoclonal antibodies, any technique that provides antibodies produced by continuous cell line cultures can be used (See, *e.g.,* Kohler and Milstein, Nature 256: 495 (1975)). Techniques for the production of single chain antibodies, as disclosed, for example, in U.S. Patent No. 4,946,778, and be used to produce single chain antibodies to polypeptides of the invention. Non-human animals may further be used to express humanized antibodies. Vaccines may comprise inoculating a mammal with a Pim-2 polypeptide, or a fragment thereof, in adequate concentration to protect the animal from the inflammatory disease state, which is sought to be prevented. As polypeptides may be degraded in the gastric environment, proteinaceous vaccines are preferred to be administered parenterally.

Peptides or small molecules may be formulated in combination with a suitable pharmaceutical carrier. Carrier include, but are not limited to, saline, buffered saline, water, dextrose, glycerol, ethanol and combinations thereof. Formulation of the composition, of course, will depend upon the route of administration and the physical characteristics of the active. Formulation techniques are well known in the art. Any mode of administration may be employed so long as the active elicits an effect at the inflamed tissue, such administration including, without limitation, parenteral administration (including subcutaneous, intramuscular and intraperitoneal administration), enteral administration (including oral and rectal administration), dermal and transmucal administration, and ocular and aural administration. The invention further relates to pharmaceutical packs and kits comprising one or more containers filled with an active.

## Claims

1. A method for diagnosing an inflammatory disease state using a tissue sample obtained from a patient, said method comprising the steps of:
(a) measuring a parameter indicative of the level of Pim-2, or Pim-2 mRNA, in the tissue sample of the patient;
(b) determining any difference in the measurement of said parameter in the tissue sample of the patient as compared to the measurement of said parameter in comparable tissue sample(s) obtained from one or more patients lacking the inflammatory disease state.

2. The method of claim 1, further comprising the step of:
(c) diagnosing the patient as having said inflammatory disease state when said measurement of said parameter with respect to the patient's tissue is significantly higher than in said comparable tissue sample(s) obtained from said one or more patients lacking said inflammatory disease state.

3. The method of claim 2, wherein said measurement of said parameter with respect to the patient's tissue is adjudged to be significantly higher than said measurement of said parameter in said comparable tissue sample(s) when the measurements differ more than about 50%.

4. The method of claim 2, wherein said measurement of said parameter with respect to the patient's tissue is adjudged to be significantly higher than said measurement of said parameter in said comparable tissue sample(s) when the measurements differ more than about 100%.

5. The method of claim 2, wherein said measurement of said parameter with respect to the patient's tissue is adjudged to be significantly higher than said measurement of said parameter in said comparable tissue sample(s) when the measurements differ more than about 200%.

6. The method of claim 1, wherein the parameter measured is kinase activity of Pim-2.

7. The method of claim 1, wherein the method comprises *in situ* hybridization of at least one nucleic acid probe comprising a polynucleotide sequence of at least about 15 contiguous nucleotides of SEQ ID NO:1.

8. The method of claim 7, wherein the polynucleotide sequence of said nucleic acid probe is derived includes nucleotides 294 through 311 of SEQ ID NO:1,

9. The method of claim 1, wherein the inflammatory disease state eventuates in an inflamed pancreas, tonsil, segment of the bowel, stomach lining, or thyroid.

10. A method for detecting inflammatory disease states comprising the steps of: (a) collecting a suspect sample, and (b) subjecting the suspect sample to a diagnostic test employing the nucleotide sequence of SEQ ID NO:1, or fragments thereof, said diagnostic test comprising polymerase chain reaction or nucleic acid hybridization.

11. A method for detecting inflammatory disease states comprising the steps of: collecting a suspect sample, and subjecting the sample to a diagnostic test comprising polyclonal antisera and/or monoclonal antibody raised to immunogens comprising the polypeptide sequence of SEQ ID NO:2, or immunogenic fragment thereof, said diagnostic test comprising Western blot analysis or enzyme-linked immunoassay (EL1SA).

12. A method for detecting inflammatory disease states comprising the steps of: (a) collecting a suspect sample, and subjecting the sample to a diagnostic test comprising polyclonal antisera and/or monoclonal antibody raised to immunogens comprising the polypeptide sequence of SEQ ID NO:2, or immunogenic fragment thereof, (b) detecting said polyclonal antisera and/or monoclonal antibody.

13. A screening assay for determining whether a compound would be effective in the treatment of an inflammatory disease state comprising:
(1) incubating the compound in vitro with cells that express SEQ ID NO:2, or variant thereof upon exposure to LPS;
(2) determining the extent of inhibition caused by said compound on the expression of SEQ ID NO:2, or variant thereof, by measuring a parameter indicative of the level of SEQ ID NO:2 (or variant thereof) or m-RNA translated to SEQ ID NO:2 (or variant thereof).

14. A screening assay for determining whether a compound would be effective in the treatment of an inflammatory disease state comprising:
(1) incubating *in vitro* the compound with a protein comprising SEQ ID:2, or variant thereof, having kinase activity, and a substrate with respect to said kinase activity;
(2) determining whether the compound inhibits said kinase activity of said protein with respect to said substrate.

15. The method of claim 14 wherein said protein is of recombinant origin or of natural origin.

16. A screening assay for identifying candidate compounds for the amelioration of inflammatory disease states comprising the steps of:
(a) separately cultivating a first immortalized cell line containing at least one gene of SEQ ID NO:1, or variant thereof, and a second immortalized cell line wherein the gene of SEQ ID NO:1, or variant thereof, is inactivated;
(b) subjecting both cell lines to a compound suspected of having activity in ameliorating the inflammatory disease state; and (c) determining if said compound selectively inhibits growth of said first immortalized cell line.

17. A screening assay for identifying compounds that may have use in the amelioration of inflammatory disease states due to modulation or alteration of Pim-2 activity, comprising the steps of:
(a) establishing a control system in vitro comprising Pim-2 and a substrate of Pim-2;
(b) establishing a test system in vitro comprising Pim-2, said substrate of Pim-2 and the candidate compound;
(c) measuring the activity of Pim-2 in the control and test systems; and
(d) determining that the candidate compound modulates or alters Pim-2 activity if the activity of Pim-2 in the test system is less than or greater than the activity measured for the control system.

18. A method for screening compounds for use in the treatment of inflammatory disease states comprising the steps of (a) contacting a compound with a cultured cell that expresses the *Pim-2* gene, and (b) detecting a change in the expression of the Pim-2 gene, or kinase activity of Pitn-2, in the cultured cell.

19. The method of claim 18, further comprising the step of:
(c) determining that a screened compound is useful in the treatment of inflammatory disease states when the expression of the *Pim*-2 gene, or kinase activity of Pim-2, in the cultured cell is significantly diminished by said screened compound.

20. The method of claim 19, wherein significant diminishment in step (c) is determined when the expression of the *Pim*-2 gene, or kinase activity of Pim-2, is reduced by more than about 50% by the screened compound.

21. The method of claim 19, wherein significant diminishment in step (c) is determined when the expression of the *Pim*-2 gene, or kinase activity of Pim-2, is reduced by more than about 100% by the screened compound.

22. The method of claim 19, wherein significant diminishment in step (c) is determined when the expression of the *Pim*-2 gene, or kinase activity of Pim-2, is reduced by more than about 200% by the screened compound.

23. A method for screening compounds for activity in the treatment of inflammatory disease states comprising the step of measuring the affinity of the compounds for Pim-2 in vitro.

24. Use of an antisense compound targeted to a nucleic acid sequence encoding *Pim*-2 for preparing a medicament for treating an animal having inflammatory disease.

25. Use of an oligonucleotide which specifically hybridizes to a transcript encoding human Pim-2 and suppresses the expression of the human Pim-2 for preparing a medicament for treating an inflammatory disease state.

26. Use of an antisense construct that targets Pim-2 encoding sequences for preparing a medicament for treating an inflammatory disease state in a patient.

27. The method of claims 1 to 8 in which the inflammatory disease state is inflammatory bowel disease.

28. The method of claim 27, wherein the inflammatory bowel disease to be diagnosed is Crohn's Disease or Ulcerative Colitis.

29. The method of claims 10 to 12 in which the inflammatory disease state is inflammatory bowel disease.

30. The screening assay of claims 13 to 15 in which the inflammatory disease state is inflammatory bowel disease.

31. The screening assay of claims 16 and 17 in which the inflammatory disease state is inflammatory bowel disease.

32. method of claims 18 to 22 in which the inflammatory disease states are inflammatory bowel diseases.

33. A method for screening compounds for activity in the treatment of inflammatory bowel diseases comprising measuring the affinity of the compounds for Pim-2 in vitro.

34. Use of an antibody reactive with the polypeptide of SEQ ID NO:2 for preparing a medicament for treating an individual having inflammatory bowel disease.

35. The method of claims 24 to 26 in which the inflammatory disease is inflammatory bowel disease.

## Patentansprüche

1. Verfahren zur Diagnose eines entzündlichen Krankheitszustands unter Verwendung einer von einem Patienten gewonnenen Gewebeprobe, wobei das Verfahren die folgenden Stufen umfasst:
(a) das Messen eines Parameters, der einen Hinweis auf den Gehalt an Pim-2 oder Pim-2-mRNA in der Gewebeprobe des Patienten darstellt; und
(b) das Bestimmen einer etwaigen Differenz bei der Messung dieses Parameters in der Gewebeprobe des Patienten, verglichen mit der Messung dieses Parameters in einer vergleichbaren Gewebeprobe oder in vergleichbaren Gewebeproben, die von einem oder mehreren Patienten, bei denen der entzündliche Krankheitszustand nicht vorliegt, erhalten worden sind.

2. Verfahren nach Anspruch 1, ferner umfassend die folgende Stufe:
(c) das Diagnostizieren des entzündlichen Krankheitszustands beim Patienten, wenn die Messung dieses Parameters im Patientengewebe signifikant höher ausfällt als in der oder den vergleichbaren Gewebeproben, die von einem oder mehreren Patienten, bei denen dieser entzündliche Krankheitszustand nicht vorliegt, erhalten worden sind.

3. Verfahren nach Anspruch 2, wobei die Messung des Parameters im Patientengewebe als signifikant höher im Vergleich zur Messung dieses Parameters in der oder den vergleichbaren Gewebeproben eingestuft wird, wenn die Messungen sich um mehr als etwa 50 % unterscheiden.

4. Verfahren nach Anspruch 2, wobei die Messung des Parameters im Patientengewebe als signifikant höher im Vergleich zur Messung dieses Parameters in der oder den vergleichbaren Gewebeproben eingestuft wird, wenn die Messungen sich um mehr als etwa 100 % unterscheiden.

5. Verfahren nach Anspruch 2, wobei die Messung des Parameters im Patientengewebe als signifikant höher im Vergleich zur Messung dieses Parameters in der oder den vergleichbaren Gewebeproben eingestuft wird, wenn die Messungen sich um mehr als etwa 200 % unterscheiden.

6. Verfahren nach Anspruch 1, wobei es sich bei dem gemessenen Parameter um die Kinase-Aktivität von Pim-2 handelt.

7. Verfahren nach Anspruch 1, wobei das Verfahren die in situ-Hybridisierung mindestens einer Nucleinsäuresonde umfasst, die eine Polynucleotidsequenz mit mindestens etwa 15 zusammenhängenden Nucleotiden von SEQ ID NO:1 umfasst.

8. Verfahren nach Anspruch 7, wobei die Polynucleotidsequenz der Nucleinsäuresonde die Nucleotide 294 bis 311 von SEQ ID NO:1 umfasst.

9. Verfahren nach Anspruch 1, wobei der entzündliche Krankheitszustand zur Entzündung von Pankreas, Mandeln, Darmsegmenten, Magenauskleidung oder Schilddrüse führt.

10. Verfahren zum Nachweis eines entzündlichen Krankheitszustands, umfassend die folgenden Stufen:
(a) man gewinnt eine verdächtige Probe und
(b) man unterwirft die verdächtige Probe einem diagnostischen Test unter Verwendung der Nucleotidsequenz von SEQ ID NO:1 oder von Fragmenten davon, wobei der diagnostische Test die Polymerase-Kettenreaktion oder Nucleinsäure-Hybridisierung umfasst.

11. Verfahren zum Nachweis eines entzündlichen Krankheitszustands, umfassend die folgenden Stufen: man gewinnt eine verdächtige Probe und unterwirft die Probe einem diagnostischen Test, der polyklonale Antiseren und/oder monoklonale Antikörper gegen Immunogene umfasst, die die Polypeptidsequenz von SEQ ID NO:2 oder ein immunogenes Fragment davon umfassen, wobei der diagnostische Test die Western-Blot-Analyse oder einen enzymgebundenen Immunoassay (ELISA) umfasst.

12. Verfahren zum Nachweis von entzündlichen Krankheitszuständen, umfassend die folgenden Stufen: (a) man gewinnt eine verdächtige Probe und unterwirft die Probe einem diagnostischen Test, der polyklonale Antiseren und/oder monoklonale Antikörper gegen Immunogene umfasst, die die Polypeptidsequenz von SEQ ID NO:2 oder ein immunogenes Fragment davon umfassen, und (b) man weist die polyklonalen Antiseren und/oder monoklonalen Antikörper nach.

13. Screening-Test zur Feststellung, ob eine Verbindung bei der Behandlung eines entzündlichen Krankheitszustands wirksam ist, umfassend:
(1) das in vitro-Inkubieren der Verbindung mit Zellen, die SEQ ID NO:2 oder eine Variante davon bei Einwirkung von LPS exprimieren; und
(2) das Bestimmen des durch diese Verbindung bewirkten Ausmaßes der Hemmung der Expression von SEQ ID NO:2 oder einer Variante davon durch Messen eines Parameters, der den Anteil von SEQ ID NO:2 (oder der Variante davon) oder von m-RNA, die in SEQ ID NO:2 translatiert ist (oder in eine Variante davon) anzeigt.

14. Screening-Test zur Bestimmung, ob eine Verbindung bei der Behandlung eines entzündlichen Krankheitszustands wirksam ist, umfassend:
(1) das in vitro-Inkubieren der Verbindung mit einem Protein, die dieses SEQ ID NO:2 oder eine Variante davon mit Kinase-Aktivität umfasst, und einem Substrat in bezug auf die Kinase-Aktivität;
(2) das Bestimmen, ob die Verbindung die Kinase-Aktivität des Proteins in bezug auf das Substrat hemmt.

15. Verfahren nach Anspruch 14, wobei das Protein rekombinanten Ursprungs oder natürlichen Ursprungs ist.

16. Screening-Test zum Identifizieren von als Kandidaten in Frage kommenden Verbindungen für die Besserung von entzündlichen Krankheitszuständen, umfassend die folgenden Stufen:
(a) das getrennte Züchten einer ersten immortalisierten Zelllinie, die mindestens ein Gen von SEQ ID NO:1 oder einer Variante davon umfasst, und einer zweiten immortalisierten Zelllinie, in der das Gen von SEQ ID NO:1 oder der Variante davon inaktiviert ist;
(b) das Einwirkenlassen einer Verbindung, bei der man eine Aktivität in bezug auf die Besserung des entzündlichen Krankheitszustands vermutet, auf beide Zelllinien; und
(c) das Feststellen, ob diese Verbindung ein selektives Wachstum der ersten immortalisierten Zelllinie hemmt.

17. Screening-Test zum Identifizieren von Verbindungen, die bei der Besserung von entzündlichen Krankheitszuständen aufgrund einer Modulation oder Veränderung der Pim-2-Aktivität verwendbar sein können, umfassend die folgenden Stufen:
(a) das in vitro-Bereitstellen eines Kontrollsystems, das Pim-2 und ein Substrat von Pim-2 umfasst;
(b) das in vitro-Bereitstellen eines Testsystems, das Pim-2, das Substrat von Pim-2 und die als Kandidat in Frage kommende Verbindung umfasst;
(c) das Messen der Aktivität von Pim-2 im Kontroll- und im Testsystem; und
(d) das Feststellen, dass die als Kandidat in Frage kommende Verbindung die Aktivität von Pim-2 moduliert oder verändert, wenn die Aktivität von Pim-2 im Testsystem kleiner oder größer als die für das Kontrollsystem gemessene Aktivität ist.

18. Verfahren zum Screening von Verbindungen zur Verwendung bei der Behandlung von entzündlichen Krankheitszuständen, umfassend die folgenden Stufen:
(a) das Kontaktieren einer Verbindung mit einer gezüchteten Zelle, die das Pim-2-Gen exprimiert, und (b) das Nachweisen einer Veränderung bei der Expression des Pim-2-Gens oder der Kinase-Aktivität von Pim-2 in der gezüchteten Zelle.

19. Verfahren nach Anspruch 18, ferner umfassend die folgenden Stufen:
(c) das Feststellen, dass eine dem Screening unterworfene Verbindung sich bei der Behandlung von entzündlichen Krankheitszuständen eignet, wenn die Expression des Pim-2-Gens oder der Kinase-Aktivität von Pim-2 in der gezüchteten Zelle durch die beim Screening ermittelte Verbindung signifikant verringert ist.

20. Verfahren nach Anspruch 19, wobei die signifikante Verringerung in Stufe (c) festgestellt wird, wenn die Expression des Pim-2-Gens oder der Kinase-Aktivität von Pim-2 durch die beim Screening ermittelte Verbindung um mehr als etwa 50 % verringert wird.

21. Verfahren nach Anspruch 19, wobei die signifikante Verringerung in Stufe (c) festgestellt wird, wenn die Expression des Pim-2-Gens oder der Kinase-Aktivität von Pim-2 durch die beim Screening ermittelte Verbindung um mehr als etwa 100 % verringert wird.

22. Verfahren nach Anspruch 19, wobei die signifikante Verringerung in Stufe (c) festgestellt wird, wenn die Expression des Pim-2-Gens oder der Kinase-Aktivität von Pim-2 durch die beim Screening ermittelte Verbindung um mehr als etwa 200 % verringert wird.

23. Verfahren zum Screening von Verbindungen auf Aktivität bei der Behandlung von entzündlichen Krankheitszuständen, umfassend die Stufe der in vitro-Messung der Affinität von Verbindungen für Pim-2.

24. Verwendung einer antisense-Verbindung, die auf eine Nucleinsäuresequenz, die für Pim-2 kodiert, abzielt, bei der Herstellung eines Arzneimittels zur Behandlung eines Tiers mit einer entzündlichen Krankheit.

25. Verwendung eines Oligonucleotids, das spezifisch mit einem Transkript, das für humanes Pim-2 kodiert, hybridisiert und die Expression des humanen Pim-2 unterdrückt, zur Herstellung eines Arzneimittels zur Behandlung eines entzündlichen Krankheitszustands.

26. Verwendung eines antisense-Konstrukts, das auf für Pim-2 kodierende Sequenzen abzielt, zur Herstellung eines Arzneimittels zur Behandlung eines entzündlichen Krankheitszustands bei einem Patienten.

27. Verfahren nach den Ansprüchen 1 bis 8, wobei es sich beim entzündlichen Krankheitszustand um eine entzündliche Darmerkrankung handelt.

28. Verfahren nach Anspruch 27, wobei es sich bei der zu diagnostizierenden entzündlichen Darmerkrankung um Morbus Crohn oder um ulzerative Kolitis handelt.

29. Verfahren nach den Ansprüchen 10 bis 12, wobei es sich beim entzündlichen Krankheitszustand um eine entzündliche Darmerkrankung handelt.

30. Screening-Test nach den Ansprüchen 13 bis 15, wobei es sich beim entzündlichen Krankheitszustand um eine entzündliche Darmerkrankung handelt.

31. Screening-Test nach den Ansprüchen 16 und 17, wobei es sich beim entzündlichen Krankheitszustand um eine entzündliche Darmerkrankung handelt.

32. Verfahren nach den Ansprüchen 18 bis 22, wobei es sich bei den entzündlichen Krankheitszuständen um entzündliche Darmerkrankungen handelt.

33. Verfahren zum Screening von Verbindungen auf Aktivität bei der Behandlung von entzündlichen Darmerkrankungen, umfassend das in vitro-Messen der Affinität der Verbindungen für Pim-2.

34. Verwendung eines Antikörpers, der mit dem Polypeptid von SEQ ID NO:2 reaktiv ist, zur Herstellung eines Arzneimittels zur Behandlung eines Individuums mit einer entzündlichen Darmerkrankung.

35. Verfahren nach Anspruch 24 bis 26, wobei es sich bei der entzündlichen Erkrankung um eine entzündliche Darmerkrankung handelt.

## Revendications

1. Procédé pour diagnostiquer un état de maladie inflammatoire au moyen d'un échantillon tissulaire obtenu chez un patient, ledit procédé comprenant les étapes de :
(a) mesurer un paramètre indiquant le niveau de Pim-2, ou d'ARNm de Pim-2, dans l'échantillon tissulaire du patient ;
(b) déterminer toute différence dans la mesure dudit paramètre dans l'échantillon tissulaire du patient par rapport à la mesure dudit paramètre dans un ou des échantillons tissulaires comparables obtenus chez un ou plusieurs patients exempts de l'état de maladie inflammatoire.

2. Procédé selon la revendication 1 comprenant en outre l'étape de :
(c) diagnostiquer le patient comme ayant ledit état de maladie inflammatoire quand ladite mesure dudit paramètre concernant le tissu du patient est sensiblement plus élevée que dans ledit ou lesdits échantillons tissulaires comparables obtenus chez ledit ou lesdits un ou plusieurs patients exempts dudit état de maladie inflammatoire.

3. Procédé selon la revendication 2 où ladite mesure dudit paramètre concernant le tissu du patient est jugée comme étant sensiblement plus élevée que ladite mesure dudit paramètre dans ledit ou lesdits échantillons tissulaires comparables quand les mesures diffèrent de plus d'environ 50 %.

4. Procédé selon la revendication 2 où ladite mesure dudit paramètre concernant le tissu du patient est jugée comme étant sensiblement plus élevée que ladite mesure dudit paramètre dans ledit ou lesdits échantillons tissulaires comparables quand les mesures diffèrent de plus d'environ 100 %.

5. Procédé selon la revendication 2 où ladite mesure dudit paramètre concernant le tissu du patient est jugée comme étant sensiblement plus élevée que ladite mesure dudit paramètre dans ledit ou lesdits échantillons tissulaires comparables quand les mesures diffèrent de plus d'environ 200 %.

6. Procédé selon la revendication 1 où le paramètre mesuré est l'activité kinase de Pim-2.

7. Procédé selon la revendication 1 où le procédé comprend l'hybridation *in situ* d'au moins une sonde d'acide nucléique comprenant une séquence polynucléotidique d'au moins environ 15 nucléotides contigus de SEQ ID NO : 1.

8. Procédé selon la revendication 7 où la séquence polynucléotidique de ladite sonde d'acide nucléique est dérivée inclut les nucléotides 294 à 311 de SEQ ID NO: 1.

9. Procédé selon la revendication 1 où l'état de maladie inflammatoire aboutit à un pancréas, une amygdale, un segment de l'intestin, une muqueuse de l'estomac ou une thyroïde enflammé(e).

10. Procédé pour détecter des états de maladies inflammatoires comprenant les étapes de : (a) recueillir un échantillon suspect, et (b) soumettre l'échantillon suspect à un test diagnostique employant la séquence nucléotidique de SEQ ID NO : 1, ou des fragments de celle-ci, ledit test diagnostique comprenant une réaction en chaîne par polymérase ou une hybridation d'acide nucléique.

11. Procédé pour détecter des états de maladies inflammatoires comprenant les étapes de : recueillir un échantillon suspect, et soumettre l'échantillon à un test diagnostique comprenant des antisérums polyclonaux et/ou un anticorps monoclonal dirigé(s) contre des immunogènes comprenant la séquence polypeptidique de SEQ ID NO : 2, ou un fragment immunogène de celle-ci, ledit test diagnostique comprenant une analyse par transfert de Western ou un immunoessai lié à une enzyme (ELISA).

12. Procédé pour détecter des états de maladies inflammatoires comprenant les étapes de : (a) recueillir un échantillon suspect, et soumettre l'échantillon à un test diagnostique comprenant des antisérums polyclonaux et/ou un anticorps monoclonal dirigé(s) contre des immunogènes comprenant la séquence polypeptidique de SEQ ID NO : 2, ou un fragment immunogène de celle-ci, (b) détecter lesdits antisérums polyclonaux et/ou ledit anticorps monoclonal.

13. Essai de sélection pour déterminer si un composé serait efficace dans le traitement d'un état de maladie inflammatoire comprenant :
(1) incuber le composé in vitro avec des cellules qui expriment SEQ ID NO : 2, ou un variant de celle-ci, lors d'une exposition à des LPS ;
(2) déterminer l'ampleur de l'inhibition causée par ledit composé sur l'expression de SEQ ID NO : 2, ou un variant de celle-ci, en mesurant un paramètre indiquant le niveau de SEQ ID NO : 2 (ou un variant de celle-ci) ou d'ARNm traduit en SEQ ID NO : 2 (ou un variant de celle-ci).

14. Essai de sélection pour déterminer si un composé serait efficace dans le traitement d'un état de maladie inflammatoire comprenant :
(1) incuber *in vitro* le composé avec une protéine comprenant SEQ ID NO : 2, ou un variant de celle-ci, ayant une activité kinase, et un substrat concernant ladite activité kinase;
(2) déterminer si le composé inhibe ladite activité kinase de ladite protéine concernant ledit substrat.

15. Procédé selon la revendication 14 où ladite protéine est d'origine recombinante ou d'origine naturelle.

16. Essai de sélection pour identifier des composés candidats pour l'amélioration d'états de maladies inflammatoires comprenant les étapes de :
(a) cultiver séparément une première lignée cellulaire immortalisée contenant au moins un gène de SEQ ID NO : 1, ou un variant de celui-ci, et une seconde lignée cellulaire immortalisée où le gène de SEQ ID NO : 1, ou un variant de celui-ci, est inactivé ;
(b) soumettre les deux lignées cellulaires à un composé suspecté d'avoir une activité dans l'amélioration de l'état de maladie inflammatoire ; et (c) déterminer si ledit composé inhibe sélectivement la croissance de ladite première lignée cellulaire immortalisée.

17. Essai de sélection pour identifier des composés qui peuvent avoir une utilité dans l'amélioration d'états de maladies inflammatoires du fait d'une modulation ou altération de l'activité de Pim-2, comprenant les étapes de :
(a) établir un système témoin in vitro comprenant Pim-2 et un substrat de Pim-2 ;
(b) établir un système de test in vitro comprenant Pim-2, ledit substrat de Pim-2 et le composé candidat ;
(c) mesurer l'activité de Pim-2 dans les systèmes témoin et de test ; et
(d) déterminer que le composé candidat module ou altère l'activité de Pim-2 si l'activité de Pim-2 dans le système de test est inférieure ou supérieure à l'activité mesurée pour le système témoin.

18. Procédé pour sélectionner des composés destinés à être utilisés dans le traitement d'états de maladies inflammatoires comprenant les étapes de : (a) mettre en contact un composé avec une cellule cultivée qui exprime le gène *Pim-2,* et (b) détecter un changement dans l'expression du gène *Pim-2,* ou l'activité kinase de Pim-2, dans la cellule cultivée.

19. Procédé selon la revendication 18 comprenant en outre l'étape de :
(c) déterminer qu'un composé sélectionné est utile dans le traitement d'états de maladies inflammatoires quand l'expression du gène *Pim-2,* ou l'activité kinase de Pim-2, dans la cellule cultivée est diminuée sensiblement par ledit composé sélectionné.

20. Procédé selon la revendication 19 où une diminution sensible dans l'étape (c) est déterminée quand l'expression du gène *Pim-2,* ou l'activité kinase de Pim-2, est réduite de plus d'environ 50 % par le composé sélectionné.

21. Procédé selon la revendication 19 où une diminution sensible dans l'étape (c) est déterminée quand l'expression du gène *Pim-2,* ou l'activité kinase de Pim-2, est réduite de plus d'environ 100 % par le composé sélectionné.

22. Procédé selon la revendication 19 où une diminution sensible dans l'étape (c) est déterminée quand l'expression du gène *Pim-2,* ou l'activité kinase de Pim-2, est réduite de plus d'environ 200 % par le composé sélectionné.

23. Procédé pour sélectionner des composés pour une activité dans le traitement d'états de maladies inflammatoires comprenant l'étape de mesure de l'affinité des composés pour Pim-2 in vitro.

24. Utilisation d'un composé antisens amené à viser une séquence d'acide nucléique codant *Pim-2* pour préparer un médicament pour traiter un animal ayant une maladie inflammatoire.

25. Utilisation d'un oligonucléotide qui s'hybride spécifiquement à un transcrit codant Pim-2 humaine et supprime l'expression de Pim-2 humaine pour préparer un médicament pour traiter un état de maladie inflammatoire.

26. Utilisation d'une construction antisens qui vise des séquences codant Pim-2 pour préparer un médicament pour traiter un état de maladie inflammatoire chez un patient.

27. Procédé selon les revendications 1 à 8 où l'état de maladie inflammatoire est une maladie intestinale inflammatoire.

28. Procédé selon la revendication 27 où la maladie intestinale inflammatoire destinée à être diagnostiquée est la maladie de Crohn ou la colite ulcéreuse.

29. Procédé selon les revendications 10 à 12 où l'état de maladie inflammatoire est une maladie intestinale inflammatoire.

30. Essai de sélection selon les revendications 13 à 15 où l'état de maladie inflammatoire est une maladie intestinale inflammatoire.

31. Essai de sélection selon les revendications 16 et 17 où l'état de maladie inflammatoire est une maladie intestinale inflammatoire.

32. Procédé selon les revendications 18 à 22 où les états de maladies inflammatoires sont des maladies intestinales inflammatoires.

33. Procédé pour sélectionner des composés pour une activité dans le traitement de maladies intestinales inflammatoires comprenant la mesure de l'affinité des composés pour Pim-2 in vitro.

34. Utilisation d'un anticorps réactif avec le polypeptide de SEQ ID NO : 2 pour préparer un médicament pour traiter un individu ayant une maladie intestinale inflammatoire.

35. Procédé selon les revendications 24 à 26 où la maladie inflammatoire est une maladie intestinale inflammatoire.


### SEQUENCE LISTING

<110> Boehringer Ingelheim Pharmaceuticals, Inc.
<120> Methods and Compounds for the Diagnosis of Inflammatory Disease and Identification of Pharmacological Agents Useful in the Treatment of Inflammatory Disease
<130> 9/206-217
<140> To Be Determined
   <141> 2002-05-23
<150> US 60/292,968
   <151> 2001-05-23
<160> 7
<170> Patent In version 3.1
<210> 1
   <211> 1535
   <212> DNA
   <213> Homo sapiens
   <220>
   <221> CDS
   <222> (185)..(1117)
   <223>
<400> 1
   ggacgcgtgg gcgcgcgcgg cgaatctcaa cgctgcgccg tctgcgggcg cttccgggcc      60
   accagtttct ctgctttcca ccctggcgcc ccccagccct ggctccccag ctgcgctgcc     120
   ccgggcgtcc acgccctgcg ggcttagcgg gttcagtggg ctcaatctgc gcagcgccac     180
   ctcc atg ttg acc aag cct cta cag ggg cct ccc gcg ccc ccc ggg acc     229
        Met Leu Thr Lys Pro Leu Gln Gly Pro Pro Ala Pro Pro Gly Thr
        1               5                   10                  15
   ccc acg ccg ccg cca gga ggc aag gat cgg gaa gcg ttc gag gcc gag      277
   Pro Thr Pro Pro Pro Gly Gly Lys Asp Arg Glu Ala Phe Glu Ala Glu
                   20                  25                  30
   tat cga ctc ggc ccc ctc ctg ggt aag ggg ggc ttt ggc acc gtc ttc      325
   Tyr Arg Leu Gly Pro Leu Leu Gly Lys Gly Gly Phe Gly Thr Val Phe
               35                  40                  45
   gca gga cac cgc ctc aca gat cga ctc cag gtg gcc atc aaa gtg att      373
   Ala Gly His Arg Leu Thr Asp Arg Leu Gln Val Ala Ile Lys Val Ile
           50                  55                  60
   ccc cgg aat cgt gtg ctg ggc tgg tcc ccc ttg tca gac tca gtc aca      421
   Pro Arg Asn Arg Val Leu Gly Trp Ser Pro Leu Ser Asp Ser Val Thr
       65                  70                  75
   tgc cca ctc gaa gtc gca ctg cta tgg aaa gtg ggt gca ggt ggt ggg      469
   Cys Pro Leu Glu Val Ala Leu Leu Trp Lys Val Gly Ala Gly Gly Gly
   80                  85                  90                  95
   cac cct ggc gtg atc cgc ctg ctt gac tgg ttt gag aca cag gag ggc      517
   His Pro Gly Val Ile Arg Leu Leu Asp Trp Phe Glu Thr Gln Glu Gly
                   100                 105                 110
   ttc atg ctg gtc ctc gag cgg cct ttg ccc gcc cag gat ctc ttt gac      565
   Phe Met Leu Val Leu Glu Arg Pro Leu Pro Ala Gln Asp Leu Phe Asp
               115                 120                 125
   tat atc aca gag aag ggc cca ctg ggt gaa ggc cca agc cgc tgc ttc      613
   Tyr Ile Thr Glu Lys Gly Pro Leu Gly Glu Gly Pro Ser Arg Cys Phe
           130                 135                 140
   ttt ggc caa gta gtg gca gcc atc cag cac tgc cat tcc cgt gga gtt      661
   Phe Gly Gln Val Val Ala Ala Ile Gln His Cys His Ser Arg Gly Val
       145                 150                 155
   gtc cat cgt gac atc aag gat gag aac atc ctg ata gac cta cgc cgt      709
   Val His Arg Asp Ile Lys Asp Glu Asn Ile Leu Ile Asp Leu Arg Arg
   160                 165                 170                 175
   ggc tgt gcc aaa ctc att gat ttt ggt tct ggt gcc ctg ctt cat gat      757
   Gly Cys Ala Lys Leu Ile Asp Phe Gly Ser Gly Ala Leu Leu His Asp
                   180                 185                 190
   gaa ccc tac act gac ttt gat ggg aca agg gtg tac agc ccc cca gag      805
   Glu Pro Tyr Thr Asp Phe Asp Gly Thr Arg Val Tyr Ser Pro Pro Glu
               195                 200                 205
   tgg atc tct cga cac cag tac cat gca ctc ccg gcc act gtc tgg tca      853
   Trp Ile Ser Arg His Gln Tyr His Ala Leu Pro Ala Thr Val Trp Ser
           210                 215                 220
   ctg ggc atc ctc ctc tat gac atg gtg tgt ggg gac att ccc ttt gag      901
   Leu Gly Ile Leu Leu Tyr Asp Met Val Cys Gly Asp Ile Pro Phe Glu
       225                 230                 235
   agg gac cag gag att ctg gaa gct gag ctc cac ttc cca gcc cat gtc      949
   Arg Asp Gln Glu Ile Leu Glu Ala Glu Leu His Phe Pro Ala His Val
   240                 245                 250                 255
   tcc cca gac tgc tgt gcc cta atc cgc cgg tgc ctg gcc ccc aaa cct      997
   Ser Pro Asp Cys Cys Ala Leu Ile Arg Arg Cys Leu Ala Pro Lys Pro
                   260                 265                 270
   tct tcc cga ccc tca ctg gaa gag atc ctg ctg gac ccc tgg atg caa     1045
   Ser Ser Arg Pro Ser Leu Glu Glu Ile Leu Leu Asp Pro Trp Met Gln
               275                 280                 285
   aca cca gcc gag gat gta ccc ctc aac ccc tcc aaa gga ggc cct gcc     1093
   Thr Pro Ala Glu Asp Val Pro Leu Asn Pro Ser Lys Gly Gly Pro Ala
           290                 295                 300
   cct ttg gcc tgg tcc ttg cta ccc taagcctggc ctggcctggc ctggccccca    1147
   Pro Leu Ala Trp Ser Leu Leu Pro
       305                 310
   atggtcagaa gagccatccc atggccatgt cacagggata gatggacatt tgttgacttg   1207
   gttttacagg tcattaccag tcattaaagt ccagtattac taaggtaagg gattgaggat 1267
   caggggttag aagacataaa ccaagtctgc ccagttccct tcccaatcct acaaaggagc 1327
   cttcctccca gaacctgtgg tccctgattc tggaggggga acttcttgct tctcattttg 1387
   ctaaggaagt ttattttggt gaagttgttc ccattctgag ccccgggact cttattctga 1447
   tgatgtgtca ccccacattg gcacctccta ctaccaccac acaaacttag ttcatatgct 1507
   cttacttggg caagggtgct ttccttcc 1535
<210> 2
   <211> 311
   <212> PRT
   <213> Homo sapiens
<400> 2
   Met Leu Thr Lys Pro Leu Gln Gly Pro Pro Ala Pro Pro Gly Thr Pro
   1               5                   10                  15
   Thr Pro Pro Pro Gly Gly Lys Asp Arg Glu Ala Phe Glu Ala Glu Tyr
               20                  25                  30
   Arg Leu Gly Pro Leu Leu Gly Lys Gly Gly Phe Gly Thr Val Phe Ala
           35                  40                  45
   Gly His Arg Leu Thr Asp Arg Leu Gln Val Ala Ile Lys Val Ile Pro
       50                  55                  60
   Arg Asn Arg Val Leu Gly Trp Ser Pro Leu Ser Asp Ser Val Thr Cys
   65                  70                  75                  80
   Pro Leu Glu Val Ala Leu Leu Trp Lys Val Gly Ala Gly Gly Gly His
                   85                  90                  95
   Pro Gly Val Ile Arg Leu Leu Asp Trp Phe Glu Thr Gln Glu Gly Phe
               100                 105                 110
   Met Leu Val Leu Glu Arg Pro Leu Pro Ala Gln Asp Leu Phe Asp Tyr
           115                 120                 125
   Ile Thr Glu Lys Gly Pro Leu Gly Glu Gly Pro Ser Arg Cys Phe Phe
       130                 135                 140
   Gly Gln Val Val Ala Ala Ile Gln His Cys His Ser Arg Gly Val Val
   145                 150                 155                 160
   His Arg Asp Ile Lys Asp Glu Asn Ile Leu Ile Asp Leu Arg Arg Gly
                   165                 170                 175
   Cys Ala Lys Leu Ile Asp Phe Gly Ser Gly Ala Leu Leu His Asp Glu
               180                 185                 190
   Pro Tyr Thr Asp Phe Asp Gly Thr Arg Val Tyr Ser Pro Pro Glu Trp
           195                 200                 205
   Ile Ser Arg His Gln Tyr His Ala Leu Pro Ala Thr Val Trp Ser Leu
       210                 215                 220
   Gly Ile Leu Leu Tyr Asp Met Val Cys Gly Asp Ile Pro Phe Glu Arg
   225                 230                 235                 240
   Asp Gln Glu Ile Leu Glu Ala Glu Leu His Phe Pro Ala His Val Ser
                   245                 250                 255
   Pro Asp Cys Cys Ala Leu Ile Arg Arg Cys Leu Ala Pro Lys Pro Ser
               260                 265                 270
   Ser Arg Pro Ser Leu Glu Glu Ile Leu Leu Asp Pro Trp Met Gln Thr
           275                 280                 285
   Pro Ala Glu Asp Val Pro Leu Asn Pro Ser Lys Gly Gly Pro Ala Pro
       290                 295                 300
   Leu Ala Trp Ser Leu Leu Pro
   305                 310
<210> 3
   <211> 2088
   <212> DNA
   <213> Homo sapiens
   <220>
   <221> CDS
   <222> (186)..(1187)
   <223>
<300>
   <301> Baytel, D. et al.
   <302> The human Pim-2 proto-oncogene and its testicular expression <303> Biochim. Biophys. Acta
   <304> 2
   <305> 1442
   <306> 274-285
   <307> 1998
   <308> NM_006875
   <309> 2000-11-02
   <313> (1)..(2088)
<400> 3
   gaattcggca cgagcgcgcg gcgaatctca acgctgcgcc gtctgcgggc gcttccgggc      60
   caccagtttc tctgctttcc accctggcgc cccccagccc tggctcccca gctgcgctgc     120
   cccgggcgtc cacgccctgc gggcttagcg ggttcagtgg gctcaatctg cgcagcgcca     180
   cctcc atg ttg acc aag cct cta cag ggg cct ccc gcg ccc ccc ggg acc     230
         Met Leu Thr Lys Pro Leu Gln Gly Pro Pro Ala Pro Pro Gly Thr
         1               5                   10                  15
   ccc acg ccg ccg cca gga ggc aag gat cgg gaa gcg ttc gag gcc gag      278
   Pro Thr Pro Pro Pro Gly Gly Lys Asp Arg Glu Ala Phe Glu Ala Glu
                   20                  25                  30
   tat cga ctc ggc ccc ctc ctg ggt aag ggg ggc ttt ggc acc gtc ttc      326
   Tyr Arg Leu Gly Pro Leu Leu Gly Lys Gly Gly Phe Gly Thr Val Phe
               35                  40                  45
   gca gga cac cgc ctc aca gat cga ctc cag gtg gcc atc aaa gtg att      374
   Ala Gly His Arg Leu Thr Asp Arg Leu Gln Val Ala Ile Lys Val Ile
           50                  55                  60
   ccc cgg aat cgt gtg ctg ggc tgg tcc ccc ttg tca gac tca gtc aca      422
   Pro Arg Asn Arg Val Leu Gly Trp Ser Pro Leu Ser Asp Ser Val Thr
       65                  70                  75
   tgc cca ctc gaa gtc gca ctg cta tgg aaa gtg ggt gca ggt ggt ggg      470
   Cys Pro Leu Glu Val Ala Leu Leu Trp Lys Val Gly Ala Gly Gly Gly
   80                  85                  90                  95
   cac cct ggc gtg atc cgc ctg ctt gac tgg ttt gag aca cag gaa ggc      518
   His Pro Gly Val Ile Arg Leu Leu Asp Trp Phe Glu Thr Gln Glu Gly
                   100                 105                 110
   ttc atg ctg gtc ctc gag cgg cct ttg ccc gcc cag gat ctc ttt gac      566
   Phe Met Leu Val Leu Glu Arg Pro Leu Pro Ala Gln Asp Leu Phe Asp
               115                 120                 125
   tat atc aca gag aag ggc cca ctg ggt gaa ggc cca agc cgc tgc ttc      614
   Tyr Ile Thr Glu Lys Gly Pro Leu Gly Glu Gly Pro Ser Arg Cys Phe
           130                 135                 140
   ttt ggc caa gta gtg gca gcc atc cag cac tgc cat tcc cgt gga gtt      662
   Phe Gly Gln Val Val Ala Ala Ile Gln His Cys His Ser Arg Gly Val
       145                 150                 155
   gtc cat cgt gac atc aag gat gag aac atc ctg ata gac cta cgc cgt      710
   Val His Arg Asp Ile Lys Asp Glu Asn Ile Leu Ile Asp Leu Arg Arg
   160                 165                 170                 175
   ggc tgt gcc aaa ctc att gat ttt ggt tct ggt gcc ctg ctt cat gat      758
   Gly Cys Ala Lys Leu Ile Asp Phe Gly Ser Gly Ala Leu Leu His Asp
                   180                 185                 190
   gaa ccc tac act gac ttt gat ggg aca agg gtg tac agc ccc cca gag      806
   Glu Pro Tyr Thr Asp Phe Asp Gly Thr Arg Val Tyr Ser Pro Pro Glu
               195                 200                 205
   tgg atc tct cga cac cag tac cat gca ctc ccg gcc act gtc tgg tca      854
   Trp Ile Ser Arg His Gln Tyr His Ala Leu Pro Ala Thr Val Trp Ser
           210                 215                 220
   ctg ggc atc ctc ctc tat gac atg gtg tgt ggg gac att ccc ttt gag      902
   Leu Gly Ile Leu Leu Tyr Asp Met Val Cys Gly Asp Ile Pro Phe Glu
       225                 230                 235
   agg gac cag gag att ctg gaa gct gag ctc cac ttc cca gcc cat gtc      950
   Arg Asp Gln Glu Ile Leu Glu Ala Glu Leu His Phe Pro Ala His Val
   240                 245                 250                 255
   tcc cca gac tgc tgt gcc cta atc cgc cgg tgc ctg gcc ccc aaa cct      998
   Ser Pro Asp Cys Cys Ala Leu Ile Arg Arg Cys Leu Ala Pro Lys Pro
                   260                 265                 270
   tct tcc cga ccc tca ctg gaa gag atc ctg ctg gac ccc tgg atg caa      1046
   Ser Ser Arg Pro Ser Leu Glu Glu Ile Leu Leu Asp Pro Trp Met Gln
               275                 280                 285
   aca cca gcc gag gat gtt acc cct caa ccc ctc caa agg agg ccc tgc      1094
   Thr Pro Ala Glu Asp Val Thr Pro Gln Pro Leu Gln Arg Arg Pro Cys
           290                 295                 300
   ccc ttt ggc ctg gtc ctt gct acc cta agc ctg gcc tgg cct ggc ctg      1142
   Pro Phe Gly Leu Val Leu Ala Thr Leu Ser Leu Ala Trp Pro Gly Leu
       305                 310                 315
   gcc ccc aat ggt cag aag agc cat ccc atg gcc atg tca cag gga          1187
   Ala Pro Asn Gly Gln Lys Ser His Pro Met Ala Met Ser Gln Gly
   320                 325                 330
   tagatggaca tttgttgact tggttttaca ggtcattacc agtcattaaa gtccagtatt   1247
   actaaggtaa gggattgagg atcaggggtt agaagacata aaccaagttt gcccagttcc   1307
   cttcccaatc ctacaaagga gccttcctcc cagaacctgt ggtccctgat tttggagggg   1367
   gaacttcttg cttctcattt tgctaaggaa gtttattttg gtgaagttgt tcccattttg   1427
   agccccggga ctcttatttt gatgatgtgt caccccacat tggcacctcc tactaccacc   1487
   acacaaactt agttcatatg cttttacttg ggcaagggtg ctttccttcc aataccccag   1547
   tagcttttat tttagtaaag ggaccctttc ccctagccta gggtcccata ttgggtcaag   1607
   ctgcttacct gcctcagccc aggatttttt attttggggg aggtaatgcc ctgttgttac   1667
   cccaaggctt cttttttttt tttttttttt ttgggtgagg ggaccctact ttgttatccc   1727
   aagtgctctt attctggtga gaagaacctt aattccataa tttgggaagg aatggaagat   1787
   ggacaccacc ggacaccacc agacaatagg atgggatgga tggttttttg ggggatgggc   1847
   taggggaaat aaggcttgct gtttgttttc ctggggcgct ccctccaatt ttgcagattt   1907
   ttgcaacctc ctcctgagcc gggattgtcc aattactaaa atgtaaataa tcacgtattg   1967
   tggggagggg agttccaagt gtgccctcct tttttttcct gcctggatta tttaaaaagc   2027
   catgtgtgga aacccactat ttaataaaag taatagaatc agaaaaaaaa aaaaaaaaaa 2087
   a 2088
<210> 4
   <211> 334
   <212> PRT
   <213> Homo sapiens
<400> 4
   Met Leu Thr Lys Pro Leu Gln Gly Pro Pro Ala Pro Pro Gly Thr Pro
   1 5 10 15
   Thr Pro Pro Pro Gly Gly Lys Asp Arg Glu Ala Phe Glu Ala Glu Tyr
   20 25 30
   Arg Leu Gly Pro Leu Leu Gly Lys Gly Gly Phe Gly Thr Val Phe Ala
   35 40 45
   Gly His Arg Leu Thr Asp Arg Leu Gln Val Ala Ile Lys Val Ile Pro
   50 55 60
   Arg Asn Arg Val Leu Gly Trp Ser Pro Leu Ser Asp Ser Val Thr Cys
   65 70 75 80
   Pro Leu Glu Val Ala Leu Leu Trp Lys Val Gly Ala Gly Gly Gly His
   85 90 95
   Pro Gly Val Ile Arg Leu Leu Asp Trp Phe Glu Thr Gln Glu Gly Phe
   100 105 110
   Met Leu Val Leu Glu Arg Pro Leu Pro Ala Gln Asp Leu Phe Asp Tyr
   115 120 125
   Ile Thr Glu Lys Gly Pro Leu Gly Glu Gly Pro Ser Arg Cys Phe Phe
   130 135 140
   Gly Gln Val Val Ala Ala Ile Gln His Cys His Ser Arg Gly Val Val
   145 150 155 160
   His Arg Asp Ile Lys Asp Glu Asn Ile Leu Ile Asp Leu Arg Arg Gly
   165 170 175
   Cys Ala Lys Leu Ile Asp Phe Gly Ser Gly Ala Leu Leu His Asp Glu
   180 185 190
   Pro Tyr Thr Asp Phe Asp Gly Thr Arg Val Tyr Ser Pro Pro Glu Trp
   195 200 205
   Ile Ser Arg His Gln Tyr His Ala Leu Pro Ala Thr Val Trp Ser Leu
   210 215 220
   Gly Ile Leu Leu Tyr Asp Met Val Cys Gly Asp Ile Pro Phe Glu Arg
   225 230 235 240
   Asp Gln Glu Ile Leu Glu Ala Glu Leu His Phe Pro Ala His Val Ser
   245 250 255
   Pro Asp Cys Cys Ala Leu Ile Arg Arg Cys Leu Ala Pro Lys Pro Ser
   260 265 270
   Ser Arg Pro Ser Leu Glu Glu Ile Leu Leu Asp Pro Trp Met Gln Thr
   275 280 285
   Pro Ala Glu Asp Val Thr Pro Gln Pro Leu Gln Arg Arg Pro Cys Pro
   290 295 300
   Phe Gly Leu Val Leu Ala Thr Leu Ser Leu Ala Trp Pro Gly Leu Ala
   305 310 315 320
   Pro Asn Gly Gln Lys Ser His Pro Met Ala Met Ser Gln Gly
   325 330
<210> 5
   <211> 311
   <212> PRT
   <213> Homo sapiens
   <220>
   <221> PEPTIDE
   <222> (1)..(311)
   <223>
<300>
   <308> AB042425
   <309> 2000-05-11
   <313> (1)..(311)
<400> 5
   Met Leu Thr Lys Pro Leu Gln Gly Pro Pro Ala Pro Pro Gly Thr Pro
   1               5                   10                  15
   Thr Pro Pro Pro Gly Gly Lys Asp Arg Glu Ala Phe Glu Ala Glu Tyr
               20                  25                  30
   Arg Leu Gly Pro Leu Leu Gly Lys Gly Gly Phe Gly Thr Val Phe Ala
           35                  40                  45
   Gly His Arg Leu Thr Asp Arg Leu Gln Val Ala Ile Lys Val Ile Pro
       50                  55                  60
   Arg Asn Arg Val Leu Gly Trp Ser Pro Leu Ser Asp Ser Val Thr Cys
   65                  70                  75                  80
   Pro Leu Glu Val Ala Leu Leu Trp Lys Val Gly Ala Gly Gly Gly His
                   85                  90                  95
   Pro Gly Val Ile Arg Leu Leu Asp Trp Phe Glu Thr Gln Glu Gly Phe
               100                 105                 110
   Met Leu Val Leu Glu Arg Pro Leu Pro Ala Gln Asp Leu Phe Asp Tyr
           115                 120                 125
   Ile Thr Glu Lys Gly Pro Leu Gly Glu Gly Pro Ser Arg Cys Phe Phe
       130                 135                 140
   Gly Gln Val Val Ala Ala Ile Gln His Cys His Ser Arg Gly Val Val
   145                 150                 155                 160
   His Arg Asp Ile Lys Asp Glu Asn Ile Leu Ile Asp Leu Arg Arg Gly
                   165                 170                 175
   Cys Ala Lys Leu Ile Asp Phe Gly Ser Gly Ala Leu Leu His Asp Glu
               180                 185                 190
   Pro Tyr Thr Asp Phe Asp Gly Thr Arg Val Tyr Ser Pro Pro Glu Trp
           195                 200                 205
   Ile Ser Arg His Gln Tyr His Ala Leu Pro Ala Thr Val Trp Ser Leu
       210                 215                 220
   Gly Ile Leu Leu Tyr Asp Met Val Cys Gly Asp Ile Pro Phe Glu Arg
   225                 230                 235                 240
   Asp Gln Glu Ile Leu Glu Ala Glu Leu His Phe Pro Ala His Val Ser
                   245                 250                 255
   Pro Asp Cys Cys Ala Leu Ile Arg Arg Cys Leu Ala Pro Lys Pro Ser
               260                 265                 270
   Ser Arg Pro Ser Leu Glu Glu Ile Leu Leu Asp Pro Trp Met Gln Thr
   275 280 285
   Pro Ala Glu Asp Val Pro Leu Asn Pro Ser Lys Gly Gly Pro Ala Pro
   290 295 300
   Leu Ala Trp Ser Leu Leu Pro
   305 310
<210> 6
   <211> 2055
   <212> DNA
   <213> Homo sapiens
<300>
   <308> XM_010208
   <309> 2001-04-17
   <313> (1) .. (2055)
<400> 6
   gcgcgcggcg aatctcaacg ctgcgccgtc tgcgggcgct tccgggccac cagtttctct     60
   gctttccacc ctggcgcccc ccagccctgg ctccccagct gcgctgcccc gggcgtccac    120
   gccctgcggg cttagcgggt tcagtgggct caatctgcgc agcgccacct ccatgttgac    180
   caagcctcta caggggcctc ccgcgccccc cgggaccccc acgccgccgc caggaggcaa    240
   ggatcgggaa gcgttcgagg ccgagtatcg actcggcccc ctcctgggta aggggggctt    300
   tggcaccgtc ttcgcaggac accgcctcac agatcgactc caggtggcca tcaaagtgat    360
   tccccggaat cgtgtgctgg gctggtcccc cttgtcagac tcagtcacat gcccactcga    420
   agtcgcactg ctatggaaag tgggtgcagg tggtgggcac cctggcgtga tccgcctgct    480
   tgactggttt gagacacagg agggcttcat gctggtcctc gagcggcctt tgcccgccca    540
   ggatctcttt gactatatca cagagaaggg cccactgggt gaaggcccaa gccgctgctt    600
   ctttggccaa gtagtggcag ccatccagca ctgccattcc cgtggagttg tccatcgtga    660
   catcaaggat gagaacatcc tgatagacct acgccgtggc tgtgccaaac tcattgattt    720
   tggttctggt gccctgcttc atgatgaacc ctacactgac tttgatggga caagggtgta    780
   cagcccccca gagtggatct ctcgacacca gtaccatgca ctcccggcca ctgtctggtc    840
   actgggcatc ctcctctatg acatggtgtg tggggacatt ccctttgaga gggaccagga    900
   gattctggaa gctgagctcc acttcccagc ccatgtctcc ccagactgct gtgccctaat    960
   ccgccggtgc ctggccccca aaccttcttc ccgaccctca ctggaagaga tcctgctgga   1020
   cccctggatg caaacaccag ccgaggatgt acccctcaac cctccaaagg gaggccctgc   1080
   ccctttggcc tggtccttgc taccctaagc ctggcctggc ctggcctggc ccccaatggt 1140
   cagaagagcc atcccatggc catgtcacag ggatagatgg acatttgttg acttggtttt 1200
   acaggtcatt accagtcatt aaagtccagt attactaagg taagggattg aggatcaggg 1260
   gttagaagac ataaaccaag tctgcccagt tcccttccca atcctacaaa ggagccttcc 1320
   tcccagaacc tgtggtccct gattctggag ggggaacttc ttgcttctca ttttgctaag 1380
   gaagtttatt ttggtgaagt tgttcccatt ctgagccccg ggactcttat tctgatgatg 1440
   tgtcacccca cattggcacc tcctactacc accacacaaa cttagttcat atgctcttac 1500
   ttgggcaagg gtgctttcct tccaataccc cagtagcttt tattttagta aagggaccct 1560
   ttcccctagc ctagggtccc atattgggtc aagctgctta cctgcctcag cccaggattc 1620
   tttattctgg gggaggtaat gccctgttgt taccccaagg cttctttttt tttttttttt 1680
   ttttgggtga ggggacccta ctctgttatc ccaagtgctc ttattctggt gagaagaacc 1740
   ttacttccat aatttgggaa ggaatggaag atggacacca ccggacacca ccagacacta 1800
   ggatgggatg gatggttttt tgggggatgg gctaggggaa ataaggcttg ctgtttgttc 1860
   tcctggggcg ctccctccaa cttttgcaga ttcttgcaac ctcctcctga gccgggattg 1920
   tccaattact aaaatgtaaa taatcacgta ttgtggggag gggagttcca agtgtgccct 1980
   cctctcttct cctgcctgga ttatttaaaa agccatgtgt ggaaacccac tatttaataa 2040
   aagtaataga atcag 2055
<210> 7
   <211> 311
   <212> PRT
   <213> Homo sapiens
<300>
   <308> XP_010208
   <309> 2001-07-12
   <313> (1)..(311)
<400> 7
   Met Leu Thr Lys Pro Leu Gln Gly Pro Pro Ala Pro Pro Gly Thr Pro
   1               5                   10                  15
   Thr Pro Pro Pro Gly Gly Lys Asp Arg Glu Ala Phe Glu Ala Glu Tyr
               20                  25                  30
   Arg Leu Gly Pro Leu Leu Gly Lys Gly Gly Phe Gly Thr Val Phe Ala
           35                  40                  45
   Gly His Arg Leu Thr Asp Arg Leu Gln Val Ala Ile Lys Val Ile Pro
        50                  55                  60
   Arg Asn Arg Val Leu Gly Trp Ser Pro Leu Ser Asp Ser Val Thr Cys
   65                  70                  75                  80
   Pro Leu Glu Val Ala Leu Leu Trp Lys Val Gly Ala Gly Gly Gly His
                   85                  90                  95
   Pro Gly Val Ile Arg Leu Leu Asp Trp Phe Glu Thr Gln Glu Gly Phe
               100                 105                 110
   Met Leu Val Leu Glu Arg Pro Leu Pro Ala Gln Asp Leu Phe Asp Tyr
           115                 120                 125
   Ile Thr Glu Lys Gly Pro Leu Gly Glu Gly Pro Ser Arg Cys Phe Phe
       130                 135                 140
   Gly Gln Val Val Ala Ala Ile Gln His Cys His Ser Arg Gly Val Val
   145                 150                 155                 160
   His Arg Asp Ile Lys Asp Glu Asn Ile Leu Ile Asp Leu Arg Arg Gly
                   165                 170                 175
   Cys Ala Lys Leu Ile Asp Phe Gly Ser Gly Ala Leu Leu His Asp Glu
               180                 185                 190
   Pro Tyr Thr Asp Phe Asp Gly Thr Arg Val Tyr Ser Pro Pro Glu Trp
           195                 200                 205
   Ile Ser Arg His Gln Tyr His Ala Leu Pro Ala Thr Val Trp Ser Leu
       210                 215                 220
   Gly Ile Leu Leu Tyr Asp Met Val Cys Gly Asp Ile Pro Phe Glu Arg
   225                 230                 235                 240
   Asp Gln Glu Ile Leu Glu Ala Glu Leu His Phe Pro Ala His Val Ser
                   245                 250                 255
   Pro Asp Cys Cys Ala Leu Ile Arg Arg Cys Leu Ala Pro Lys Pro Ser
               260                 265                 270
   Ser Arg Pro Ser Leu Glu Glu Ile Leu Leu Asp Pro Trp Met Gln Thr
           275                 280                 285
   Pro Ala Glu Asp Val Pro Leu Asn Pro Ser Lys Gly Gly Pro Ala Pro
       290                 295                     300
   Leu Ala Trp Ser Leu Leu Pro
   305                 310
<210> 8
   <211> 21
   <212> RNA
   <213> Homo sapiens
<313> (1)..(21)
<400> 8
   gugauucccc ggaaucguct t 21
<210> 9
   <211> 21
   <212> RNA
   <213> Homo sapiens
<313> (1)..(21)
<400> 9
   cacgauuccg gggaaucact t 21
<210> 10
   <211> 21
   <212> RNA
   <213> Homo sapiens
<313> (1) .. (21)
<400> 10
   gugcuaaggc cccuuagugt t 21
<210> 11
   <211> 21
   <212> RNA
   <213> Homo sapiens
<313> (1) .. (21)
<400> 11
   cacuaagggg ccuuagcact t 21